(19) Europäisches Patentamt / European Patent Office / Office européen des brevets

(11) **EP 2 808 668 B1**

(12) **EUROPEAN PATENT SPECIFICATION**

(45) Date of publication and mention
of the grant of the patent:
**08.04.2020 Bulletin 2020/15**

(51) Int Cl.:
**G01N 21/15** *(2006.01)*    **G01N 21/25** *(2006.01)*
**G01N 21/85** *(2006.01)*    **G01N 33/28** *(2006.01)*
**G01N 21/94** *(2006.01)*

(21) Application number: **13741234.2**

(22) Date of filing: **23.01.2013**

(86) International application number:
**PCT/JP2013/051339**

(87) International publication number:
**WO 2013/111787 (01.08.2013 Gazette 2013/31)**

(54) **Machine, reduction gear for industrial robot and industrial robot configured to detect deterioration of lubricant**

Maschine, Untersetzungsgetriebe für Industrieroboter und Industrieroboter ausgebildet zum Erkennen von Verschleißanzeichen von Schmiermittel

Machine, réducteur de vitesse pour robot industriel et robot industriel conçus pour détecter la dégradation d'une matière lubrifiante

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2012 JP 2012013517**

(43) Date of publication of application:
**03.12.2014 Bulletin 2014/49**

(73) Proprietor: **Nabtesco Corporation**
**Tokyo 102-0093 (JP)**

(72) Inventors:
• **SHIMADA, Hideshi**
  **Tsu-shi, Mie 514-8533 (JP)**
• **NAKAMURA, Koji**
  **Tsu-shi, Mie 514-8533 (JP)**

(74) Representative: **Grünecker Patent- und Rechtsanwälte PartG mbB Leopoldstraße 4 80802 München (DE)**

(56) References cited:
WO-A1-91/00993          WO-A1-98/16822
WO-A2-03/030621         JP-A- H0 634 541
JP-A- H01 119 741       JP-A- H05 223 729
JP-A- H07 146 233       JP-A- H08 201 283
JP-A- H11 235 097       JP-A- 2005 156 297
JP-A- 2008 045 918      JP-A- 2008 215 947
JP-A- 2009 069 097      JP-A- 2010 230 412
JP-A- 2011 026 591      JP-A- 2011 122 968
US-A1- 2008 024 761     US-A1- 2009 216 464
US-A1- 2009 285 721

## Description

Technical Field

The present invention relates to a machine provided with a lubricant deterioration sensor for detecting he deterioration of lubricant.

Background Art

Conventionally, as a lubricant deterioration sensor for detecting the deterioration of lubricant of a machine, an oil deterioration degree sensor has been known. In this sensor, an oil entering gap portion for entering lubricant therein is formed on an optical path from an infrared LED (Light Emitting Diode) to a photo diode. Then, an amount of light absorption by the lubricant within the oil entering gap portion with respect to light emitted from the infrared LED is measured according to a light reception amount of the photo diode, to thereby determine a deterioration degree of the lubricant related to the light absorption amount thus measured (see patent literatures 1 and 2, for example).

However, the oil deterioration degree sensor described in each of the patent literatures 1 and 2 has a problem that, although a density of insoluble content within patent literatures 1 and 2 has a problem that, although a density of insoluble content within the lubricant can be measured as the deterioration degree of the lubricant, kinds of contaminant within the lubricant cannot be specified.

As a technique of specifying the kinds of contaminant within lubricant, a technique has been known in which an LED irradiates light toward a membrane filter having been used to filter the lubricant. Then, a light reception element converts reflection light from the contaminant on the membrane filter into digital values of RGB, to thereby specify the kinds of contaminant within the lubricant based on the RGB digital values thus converted (see non-patent literatures 1 and 2, for example).

A system, with which the composition of mud can be determined is known from US 2009/0285721 A1. In order to do so, mud is filled in a cavity of the system and therefrom guided to a measurement chamber. In the measurement chamber, two different measurement systems are provided at right angles with respect to each other, at least one system measuring the optical transmission of multi-coloured light through the chamber. To eject the mud from the measurement cell, a piston can move downwards to set an opening on the bottom of the measurement chamber free. The piston is provided with an O-ring, which serves as cleaning element for cleaning the inner circumferential surface of the measurement chamber and, thus during said movement, the optics of the sensor.

A further system, via which liquid composition may be analyzed is known from WO 98/16822. In said system, a fluid flows through a pipe into a measurement chamber and out of the measurement chamber through a further pipe. At the position, where the fluid flows through the measurement chamber, two parallel optical windows are provided on opposite sides of the chamber, to which respective light guides are coupled such as to facilitate optical transmission measurements. In the system of WO 98/16822, a cleaning element is provided, which is actuated by pressure such that it is pressed upwardly so as to clean a respective optical window of a transmission sensor while the two windows are separated by a rotational movement.

JP 2008-215987 relates to a liquid property detection device capable of removing foreign matter adhering to opposed surfaces of a light emitting element and a light receiving element forming a gap there between by a driving mechanism of a cleaning slider, thereby keeping each surface of the light emitting element and the light receiving element in a clean state during operation of an engine. The cleaning slider comprises a wiper that moves along the light emitting surface and the light incident surface. When the temperature of a lubricating oil in the gap is high and the adhesive force of a foreign matter is weak, the foreign matter adhering to the emitting surface and the incident surface is removed.

Citation List

Patent Literature

PATENT LITERATURE 1: JP-A-7-146233
PATENT LITERATURE 2: JP-A-10-104160

Non-Patent Literature

NON-PATENT LITERATURE 1: Tomohiko Yamaguchi and four others, "METHOD OF DISCRIMINATING HUE OF CONTAMINANT WITHIN LUBRICANT", Fukui University, Faculty of Engineering, Research Report, March 2003, Volume 51, No. 1, pp. 81 - 88
NON-PATENT LITERATURE 2: Tomomi Honda, "DETERIORATION DIAGNOSIS OF LUBRICANT •INSPECTION TECHNOLOGY", Journal of Japan Society for Precision Engineering, 2009, Volume 75, No. 3, pp. 359 - 362

Summary of Invention

Technical Problem

The technique described in each of the non-patent literatures 1 and 2 requires to drain lubricant from a machine and filter the lubricant by the membrane filter. Thus, there arises a problem of lacking immediacy.

Accordingly, an object of this invention is to provide a machine which can suppress the degradation of performance of a lubricant deterioration sensor that enables to immediately specify the kinds and amounts of contaminant within lubricant.

Solution to Problem

The present invention is directed to a machine as defined in claim 1.

The cleaning machine may include a plurality of insertion portions configured to be inserted in the oil gap and having elasticity. A width of each of the insertion portions in a distance direction of the oil gap is smaller than the distance of the oil gap, and an entire width of the plurality of insertion portions in the distance direction of the oil gap is larger than the distance of the oil gap.

The lubricant deterioration sensor may include: a supporting member supporting the light emitting element, the color light reception element and the gap forming member; and a fixing member configured to be fixed to the machine body. The fixing member may support the supporting member so as to be rotatable so that the direction of the opening of the oil gap changes when the supporting member rotates.

The supporting member may include a rotational driving force receiving portion at a position where the rotational driving force receiving portion does not contact with the lubricant in a state where the fixing member is fixed to the machine body. The rotational driving force receiving portion receives a rotational driving force with respect to the fixing member from outside by a contact force.

The lubricant deterioration sensor may include a rotation preventing member preventing a rotation of the supporting member with respect to the fixing member by contacting with both the supporting member and the fixing member. The rotation preventing member may include a contact driving force receiving portion at a position where the contact driving force receiving portion does not contact with the lubricant in a state where the fixing member is fixed to the machine body. The contact driving force receiving portion receives a driving force for contacting with both the supporting member and the fixing member from outside by a contact force.

The lubricant may contain molybdenum as additive.

The light emitting element may be a white LED which emits white light.

The gap forming member may have a reflection surface for bending the optical path.

The gap forming member may be configured to include two right-angle prisms each provided with the reflection surface for bending the optical path by 90 degrees, whereby the optical path is bent by 180 degrees by the reflection surfaces of the two right-angle prisms. The oil gap may be formed between the two right-angle prisms.

The supporting member may have an optical path surrounding portion which surrounds at least a part of the optical path. The optical path surrounding portion may have a surface which is treated for preventing light reflection.

The gap forming surfaces may be treated to an oil repelling treatment.

The machine may be a reduction gear for an industrial robot, and the machine body may be a main body of the reduction gear.

The machine body may include an arm and the reduction gear used at an articular portion of the arm. The lubricant may be lubricant for the reduction gear.

The arm may be the sensor side portion. The reduction gear may be the cleaning member side portion. The cleaning member may be disposed at a position contacting with the gap forming surfaces when the reduction gear rotates.

Advantageous Effects of Invention

In the lubricant deterioration sensor for the machine according to this invention, the color light reception element detects colors with respect to the light having wavelengths not absorbed by the contaminant such as ion powder within the lubricant at the oil gap among the light emitted by the light emitting element. Thus, colors of the contaminant within the lubricant can be detected immediately. That is, the lubricant deterioration sensor for the machine according to this invention enables to immediately specify the kinds and amounts of the contaminant within the lubricant based on the colors detected by the color light reception element.

Further, in the machine according to this invention, when the sensor side portion and the cleaning member side portion move relatively, the cleaning member rubs off dirt such as sludge adhered to the gap forming surfaces of the gap forming member. Thus, the degradation of performance of the lubricant deterioration sensor can be suppressed.

As a result, the machine according to this invention can suppress the degradation of performance of the lubricant deterioration sensor which enables to immediately specify the kinds and amounts of the contaminant within the lubricant.

In the machine according to this invention, as compared with a case where the cleaning member is configured only by a single insertion portion which width in the distance direction of the oil gap is larger than the distance of the oil gap, a pressure applied to the lubricant deterioration sensor by the cleaning member at the time of inserting the cleaning member in the oil gap can be mad small. As a result, the degradation of performance of the lubricant deterioration sensor due to the pressure applied to the lubricant deterioration sensor from the cleaning member can be suppressed. Further, in the machine according to this invention, dirt such as sludge adhered to the gap forming surfaces of the gap forming member can be effectively rubbed off by the plurality of insertion portions of the cleaning member.

In the machine according to this invention, the direction of the opening of the oil gap in the case of fixing the fixing member to the machine body can be adjusted so that the dirt cleaning effects of the cleaning member in the case of fixing the fixing member to the machine body can be enhanced.

In the machine according to this invention, the direction of the opening of the oil gap in the case of fixing the fixing member to the machine body can be adjusted so that the dirt cleaning effects of the cleaning member after fixing the fixing member to the machine body can be enhanced.

In the machine according to this invention, the direction of the opening of the oil gap in the case of fixing the fixing member to the machine body can be fixed so that the dirt cleaning effects of the cleaning member after fixing the fixing member to the machine body can be enhanced.

In the machine according to this invention, since molybdenum can be prevented from adhering to the gap forming member as sludge, the degradation of the performance of the lubricant deterioration sensor can be suppressed.

In the machine according to this invention, the lubricant deterioration sensor can be miniaturized as compared with a configuration where the light emitting element is a lamp other than an LED, for example. Thus, the machine according to this invention can be miniaturized.

In the machine according to this invention, as compared with a configuration where the optical path from the light emitting element to the color light reception element is straight, the lubricant deterioration sensor can be miniaturized by disposing the light emitting element and the color light reception element close to each other. Further, in the machine according to this invention, the gap forming member has a function of bending the optical path as well as a function of forming the oil gap. Thus, the number of the components of the lubricant deterioration sensor can be reduced as compared with a configuration where a member for bending the optical path is separately provided in place of the gap forming member. Therefore, the machine according to this invention can be miniaturized and further reduce the number of the components.

In the machine according to this invention, the lubricant deterioration sensor can be miniaturized with a simple configuration having a small number of the components. Thus, the machine according to this invention can be miniaturized with a simple configuration having a small number of the components.

In the machine according to this invention, the color light reception element is prevented from receiving unnecessary reflection light. Thus, the detection accuracy of the colors of the contaminant within the lubricant by the lubricant deterioration sensor can be improved as compared with a configuration where the color light reception element receives unnecessary reflection light. Thus, the machine according to this invention can improve the prediction accuracy of a failure.

The machine according to this invention is configured to easily flow the lubricant through the oil gap. Thus, the detection accuracy of the colors of the contaminant within the lubricant by the lubricant deterioration sensor can be improved as compared with a configuration where the lubricant likely remains at the oil gap. Further, in the machine according to this invention, when the surface forming the oil gap is treated to the oil repelling treatment, dirt is unlikely adhered to the surface forming the oil gap. Thus, the degradation of the detection accuracy of the colors of the contaminant within the lubricant by the lubricant deterioration sensor due to the adhesion of dirt can be suppressed. Thus, the machine according to this invention can improve the prediction accuracy of a failure.

The reducer for the industrial robot according to this invention can suppress the degradation of the performance of the lubricant deterioration sensor which enables to immediately specify the kinds and amounts of the contaminant within the lubricant. Thus, the reducer for the industrial robot according to this invention can maintain for a long time the accuracy of the immediate prediction as to a failure.

The industrial robot according to this invention can suppress the degradation of the performance of the lubricant deterioration sensor which enables to immediately specify the kinds and amounts of the contaminant within the lubricant. Thus, the industrial robot according to this invention can maintain for a long time the accuracy of the immediate prediction as to an failure.

In the industrial robot according to this invention, the cleaning member can rub off dirt such as sludge adhered to the gap forming surfaces of the lubricant deterioration sensor each time the arm is driven by the reducer.

The machine according to this invention can suppress the degradation of the performance of the lubricant deterioration sensor which enables to immediately specify the kinds and amounts of the contaminant within the lubricant.

Brief Description of Drawings

[Fig. 1] A side view of an industrial robot according to an embodiment of this invention.
[Fig. 2] A sectional view of the articular portion of the industrial robot shown in Fig. 1.
[Fig. 3] A front view of a lubricant deterioration sensor shown in Fig. 2.
[Fig. 4] A front sectional view of the lubricant deterioration sensor shown in Fig. 3 in an attached state to an arm.

[Fig. 5] Fig. 5(a) is a plan view of the lubricant deterioration sensor shown in Fig. 3. Fig. 5(b) is a bottom view of the lubricant deterioration sensor shown in Fig. 3.

[Fig. 6] Fig. 6(a) is a front view of a housing shown in Fig. 3. Fig. 6(b) is a front sectional view of the housing shown in Fig. 3.

[Fig. 7] Fig. 7(a) is a side view of the housing shown in Fig. 3. Fig. 7(b) is a side sectional view of the housing shown in Fig. 3.

[Fig. 8] Fig. 8(a) is a plan view of the housing shown in Fig. 3. Fig. 8(b) is a bottom view of the housing shown in Fig. 3.

[Fig. 9] Fig. 9(a) is a front view of a holder shown in Fig. 3. Fig. 9(b) is a front sectional view of the holder shown in Fig. 3.

[Fig. 10] Fig. 10(a) is a side view of the holder shown in Fig. 3. Fig. 10(b) is a side sectional view of the holder shown in Fig. 3.

[Fig. 11] Fig. 11(a) is a plan view of the holder shown in Fig. 3. Fig. 11(b) is a bottom view of the holder shown in Fig. 3.

[Fig. 12] A diagram showing an optical path from a white LED to an RGB sensor shown in Fig. 4.

[Fig. 13] Fig. 13(a) is a front view of a holder cap shown in Fig. 3. Fig. 13(b) is a front sectional view of the holder cap shown in Fig. 3.

[Fig. 14] Fig. 14(a) is a plan view of the holder cap shown in Fig. 3. Fig. 14(b) is a bottom view of the holder cap shown in Fig. 3.

[Fig. 15] A plan view of a component of the supporting body shown in Fig. 2 to which a cleaning member is fixed.

[Fig. 16] Fig. 16(a) is a plan view of an example of the cleaning member shown in Fig. 15. Fig. 16(b) is a plan view of another example of the cleaning member different from that shown in Fig. 16(a).

[Fig. 17] Fig. 17(a) is a plan view of the cleaning member in a case where a part of the cleaning member shown in Fig. 16(a) is inserted in an oil gap. Fig. 17(b) is a plan view of the cleaning member in a case where a part of the cleaning member shown in Fig. 16(b) is inserted in the oil gap.

[Fig. 18] A perspective view of the cleaning member in a case where an insertion portion shown in Fig. 16 is a brush.

[Fig. 19] Fig. 19(a) is a table showing the experimentation result of the chronological change of a color difference ΔE in a case where lubricant shown in Fig. 2 contains molybdenum as additive. Fig. 19(b) is a graph of the experimentation result shown in Fig. 19(a).

[Fig. 20] Fig. 20(a) is a table showing the experimentation result of the chronological change of the color difference ΔE in a case where the lubricant shown in Fig. 2 does not contain molybdenum as the additive. Fig. 20(b) is a graph of the experimentation result shown in Fig. 20(a).

[Fig. 21] A sectional diagram showing the articular portion of the industrial robot shown in Fig. 1, which shows another example different from that shown in Fig. 2.

Description of Embodiments

The inventors of the present application have developed a lubricant deterioration sensor which enables to immediately specify the kinds and amounts of contaminant within the lubricant of a machine. This lubricant deterioration sensor (hereinafter referred to as a "new sensor") is a lubricant deterioration sensor which is mounted in a machine and detects the deterioration of the lubricant of the machine. This sensor includes a light emitting element which emits light, a color light reception element which detects color of received light, and a gap forming member at which an oil gap as a gap for entering the lubricant therein is formed. The gap forming member transmits the light emitted from the light emitting element, and the oil gap is disposed on an optical path from the light emitting element to the color light reception element.

However, the inventors of the present application have found that when the new sensor is continuously mounted in the machine and used, the accuracy of the new sensor for specifying the kinds and amounts of contaminant within the lubricant degrades, that is, the performance of the new sensor degrades.

Then, the inventors of the present application have investigated a cause of the degradation of the performance of the new sensor. As a result of the investigation, the inventors of the present application have ascertained that the degradation of the performance of the new sensor is caused by a fact that sludge (sediment) generated by the deterioration of the lubricant adheres to the gap forming member.

According to this invention, there is provided with a machine which can suppress the degradation of performance of the lubricant deterioration sensor that enables to immediately specify the kinds and amounts of contaminant within lubricant.

Hereinafter, an embodiment of this invention will be explained with reference to drawings.

First, the configuration of an industrial robot as a machine according to this embodiment will be explained.

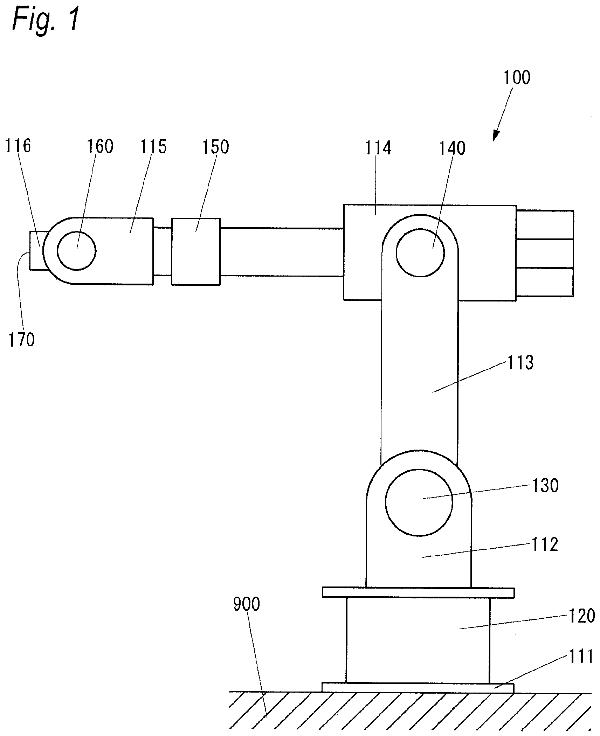

Fig. 1 is a side view of the industrial robot 100 according to this embodiment.

As shown in Fig. 1, the industrial robot 100 includes an attachment portion 111 to be attached to a mounting portion 900 such as a floor or a ceiling, arms 112 to 116, an articular portion 120 for connecting between the attachment portion 111 and the arm 112, an articular portion 130 for connecting between the arm 112 and an arm 113, an articular portion 140 for connecting between the arm 113 and an arm 114, an articular portion 150 for connecting between the arm 114 and an arm 115, an articular portion 160 for connecting between the arm 115 and an arm 116, and an articular portion 170 for connecting between the arm 116 and a not-shown hand.

Of the industrial robot 100, portions except for lubricant 131a, the lubricant deterioration sensors such as lubricant deterioration sensors 137, 139 described later and a cleaning member described later constitute a machine body according to this invention in a case where the industrial robot 100 is the machine according to this invention.

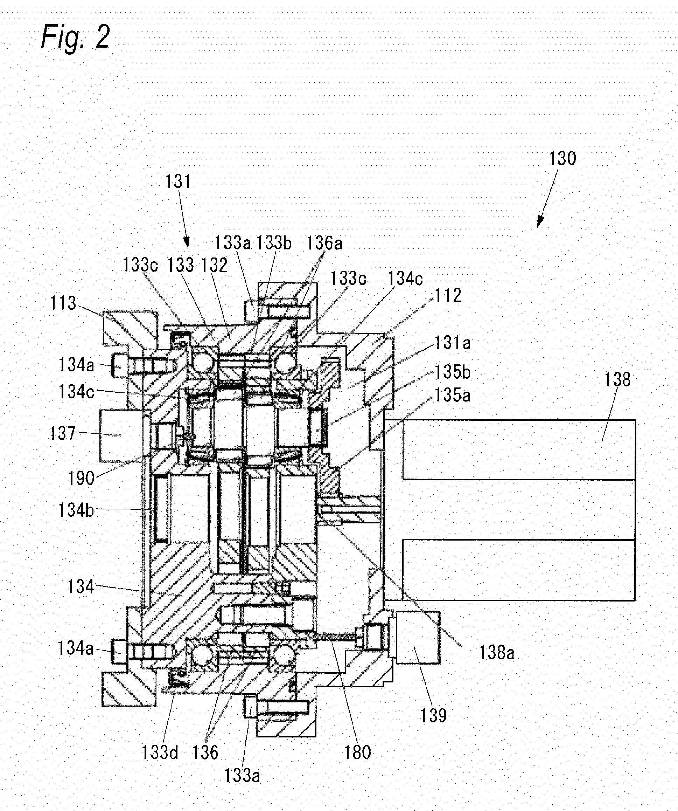

Fig. 2 is a sectional view of the articular portion 130. Although the explanation is made hereinafter as to the articular portion 130, the configuration of each of the articular portions 120, 140 to 170 is substantially same.

As shown in Fig. 2, the articular portion 130 includes a reducer 131 for connecting between the arm 112 and the arm 113, a motor 138 provided with a gear 138a at the output shaft thereof and fixed to the arm 112 by means of not-shown bolts, and the lubricant deterioration sensor 139 for detecting the deterioration of the lubricant 131a for reducing friction generated at the movable portions of the reducer 131.

The reducer 131 includes a reducer body 132 and the lubricant deterioration sensor 137 for detecting the deterioration of the lubricant 131a of the reducer body 132. The reducer body 132 constitutes a machine body according to this invention in a case where the reducer 131 is the machine according to this invention.

The reducer body 132 includes a case 133 fixed to the arm 112 by means of bolts 133a, a supporting body 134 fixed to the arm 113 by means of bolts 134a, three gears 135a which are disposed around the center axis of the reducer 131 with a constant interval therebetween and mesh with the gear 138a of the motor 138, three crank shafts 135b which are disposed around the center axis of the reducer 131 with a constant interval therebetween and fixed to the gears 135a, respectively, and two external gears 136 which mesh with internal gears 133b provided at the case 133.

The supporting body 134 is rotatably supported by the case 133 via two bearings 133c. The supporting body 134 is provided with a seal member 134b for preventing leakage of the lubricant 131a. A seal member 133d for preventing leakage of the lubricant 131a is provided between the case 133 and the supporting body 134. Cleaning members 180 for cleaning the lubricant deterioration sensor 139 are fixed to the supporting body 134.

Each of the crank shafts 135b is rotatably supported by the supporting body 134 via two bearings 134c and further rotatably supported by the external gears 136 via bearings 136a. A cleaning member 190 for cleaning the lubricant deterioration sensor 137 is fixed to the crank shaft 135b.

The lubricant deterioration sensor 137 is fixed to the support body 134. The lubricant deterioration sensor 139 is fixed to the arm 112.

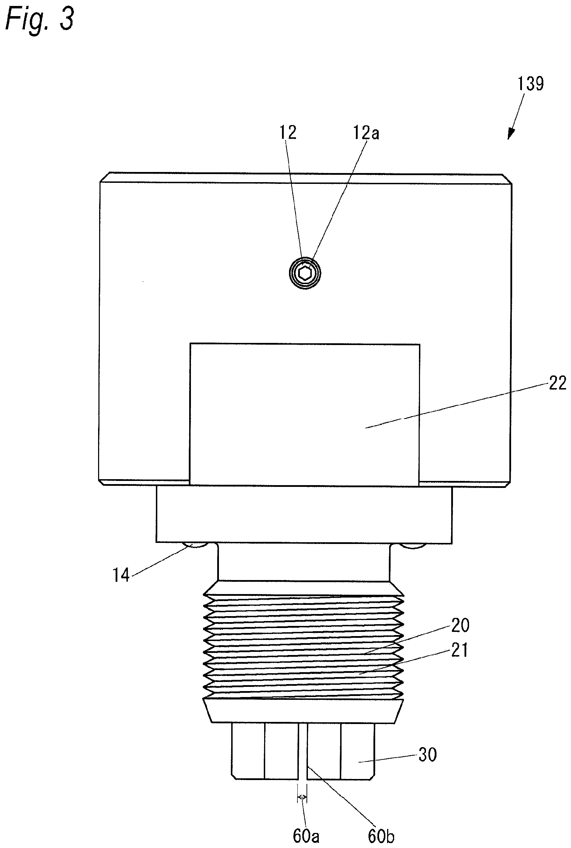

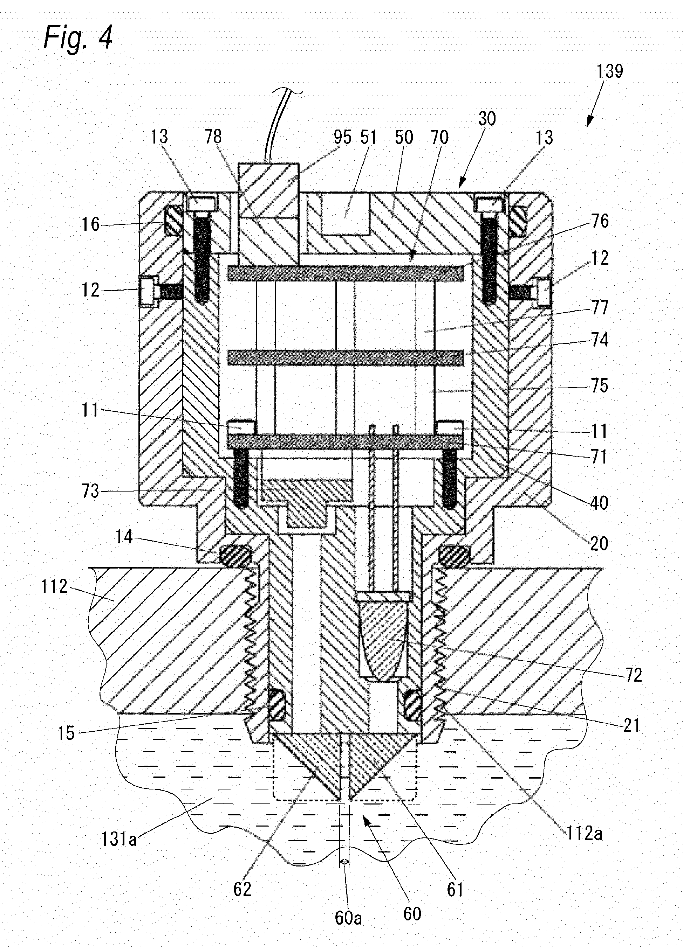

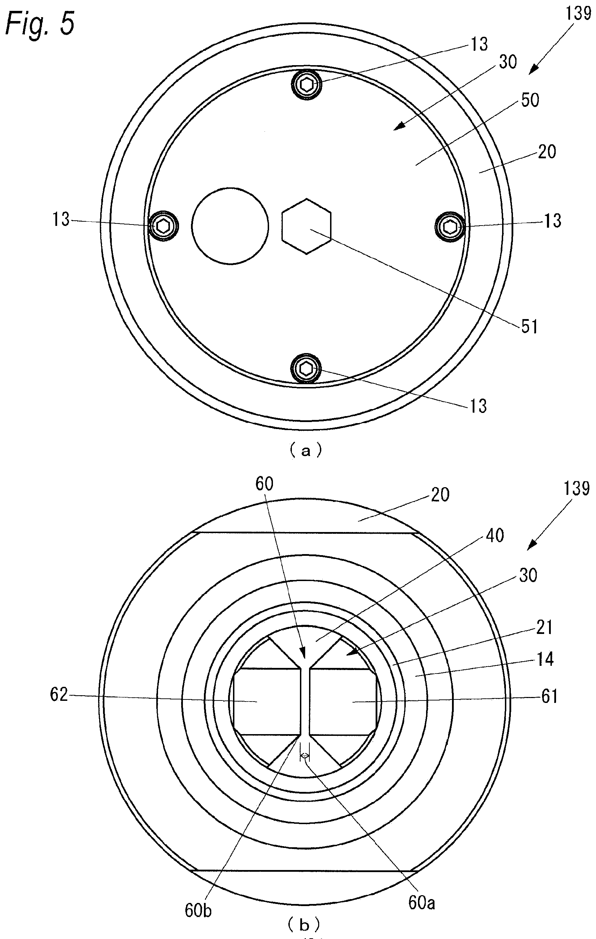

Fig. 3 is a front view of the lubricant deterioration sensor 139. Fig. 4 is a front sectional view of the lubricant deterioration sensor 139 in an attached state to the arm 112. Fig. 5(a) is a plan view of the lubricant deterioration sensor 139. Fig. 5(b) is a bottom view of the lubricant deterioration sensor 139. Although the explanation is made hereinafter as to the lubricant deterioration sensor 139, the configuration of each of the lubricant deterioration sensors such as the lubricant deterioration sensor 137 other than the lubricant deterioration sensor 139 is substantially same.

As shown in Figs. 3 to 5(b), the lubricant deterioration sensor 139 includes a housing 20 made of aluminum alloy for supporting the respective components of the lubricant deterioration sensor 139, a supporting member 30 for supporting a white LED 72, an RGB sensor 73 and a gap forming member 60 described later, the gap forming member 60 held by the supporting member 30, and an electronic component group 70 having the white LED 72 and the RGB sensor 73.

The supporting member 30 is fixed to the housing 20 by means of bolts 12 with hexagon holes. The supporting member 30 includes a holder 40 made of aluminum alloy, and a holder cap 50 made of aluminum alloy which is fixed to the holder 40 by means of bolts 13 with hexagon holes.

The gap forming member 60 is constituted by two right-angle prisms 61, 62 made of glass. An oil gap 60a as a gap for entering the lubricant 131a therein is formed between the two right-angle prisms 61, 62.

The electronic component group 70 includes a circuit board 71 fixed to the supporting member 30 by means of screws 11, the white LED 72 mounted on the circuit board 71, the RGB sensor 73 mounted on the circuit board 71, a circuit board 74 disposed at one surface side of the circuit board 71 opposing to the white LED 72 and RGB sensor 73 side thereof, a plurality of pillars 75 for fixing the circuit board 71 and the circuit board 74, a circuit board 76 disposed on the opposite surface side of the circuit board 71 with respect to the circuit board 74, a plurality of pillars 77 for fixing the circuit board 74 and the circuit board 76, and a connector 78 mounted on one surface side of the circuit board 76 opposing to the circuit board 74 side thereof. A plurality of electronic components are mounted on each of the circuit board 71, the circuit board 74 and the circuit board 76. The circuit board 71, the circuit board 74 and the circuit board 76 are mutually connected electrically.

The lubricant deterioration sensor 139 includes an O ring 14 for preventing the leakage of the lubricant 131a from a gap between the housing 20 and the arm 112, an O ring 15 for preventing the leakage of the lubricant 131a from a gap between the housing 20 and the holder 40, and an O ring 16 disposed between the housing 20 and the holder cap 50.

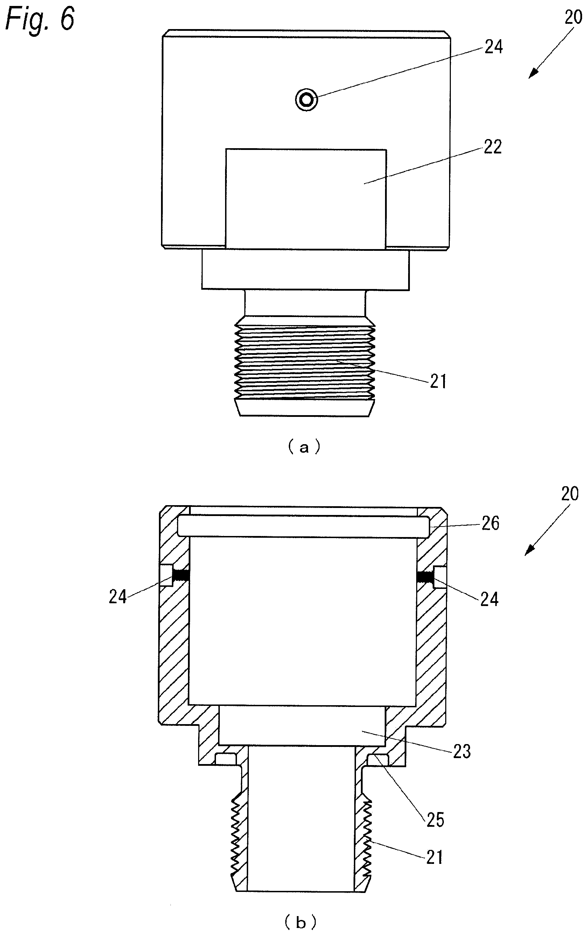

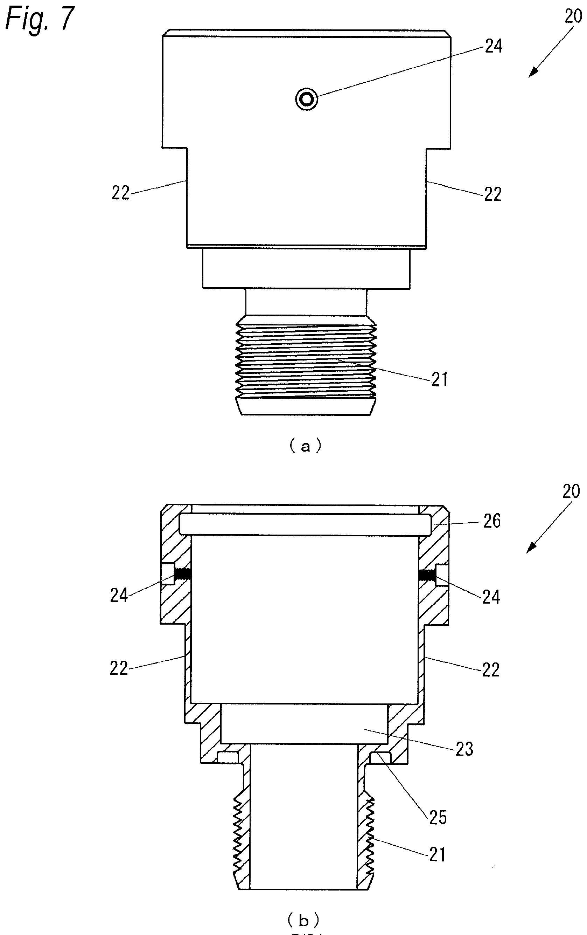

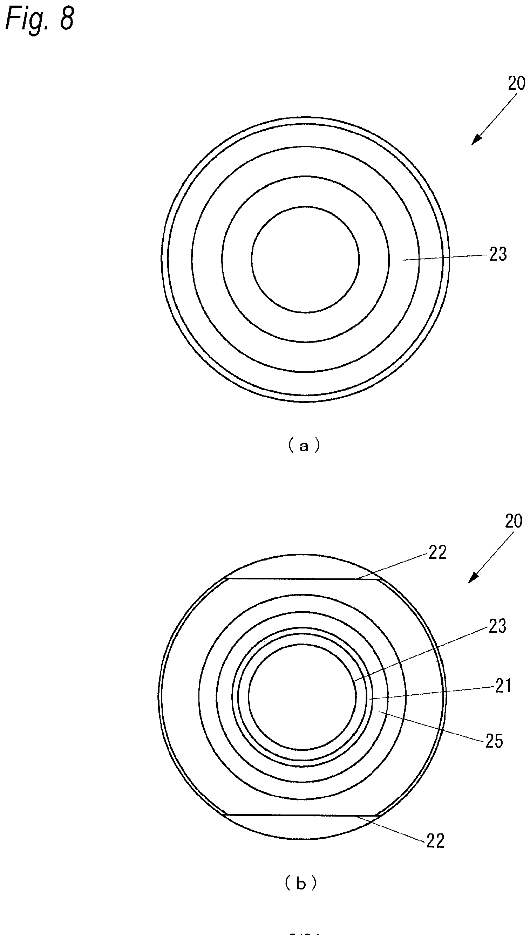

Fig. 6(a) is a front view of the housing 20. Fig. 6(b) is a front sectional view of the housing 20. Fig. 7(a) is a side view of the housing 20. Fig. 7(b) is a side sectional view of the housing 20. Fig. 8(a) is a plan view of the housing 20. Fig. 8(b) is a bottom view of the housing 20.

As shown in Figs. 3 to 8, the housing 20 includes a screw portion 21 to be fixed to the screw hole 112a of the arm 112, a tool contact portion 22 which is grasped by a tool such as a wrench at the time of rotating the screw portion 21 with respect to the screw hole 112a of the arm 112, and a holder housing portion 23 in which the holder 40 is housed. Further, the housing 20 is provided with screw holes 24 into which the bolts 12 with hexagon holes are respectively screwed, a groove 25 into which the O ring 14 is fit, and a groove 26 into which the O ring 16 is fit. The housing 20 is configured to be fixed to the arm 112 of the industrial robot 100, that is, the machine body and hence constitutes a fixing member of this invention.

The screw hole 112a of the arm 112 may be used for supplying the lubricant 131a to the reducer 131 and for disposing the lubricant 131a from the reducer 131 in a state that the lubricant deterioration sensor 139 is removed.

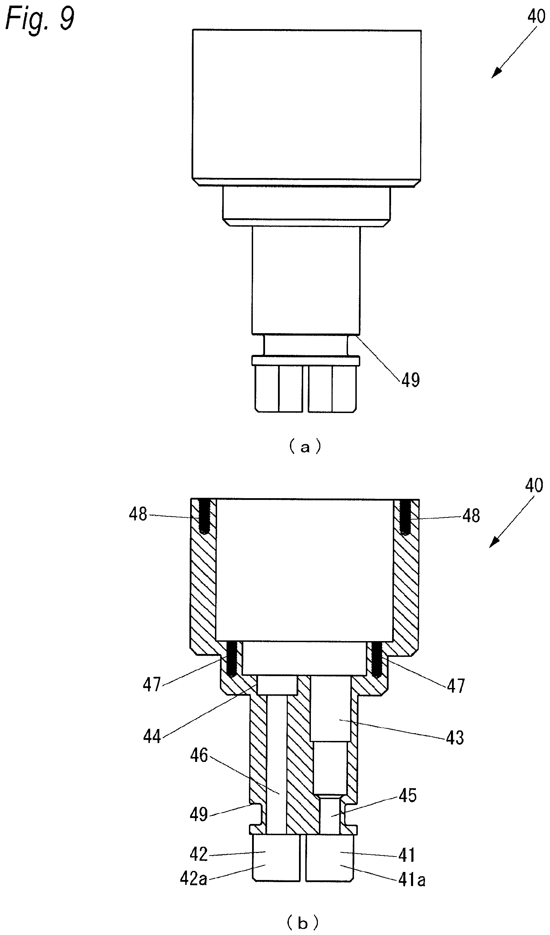

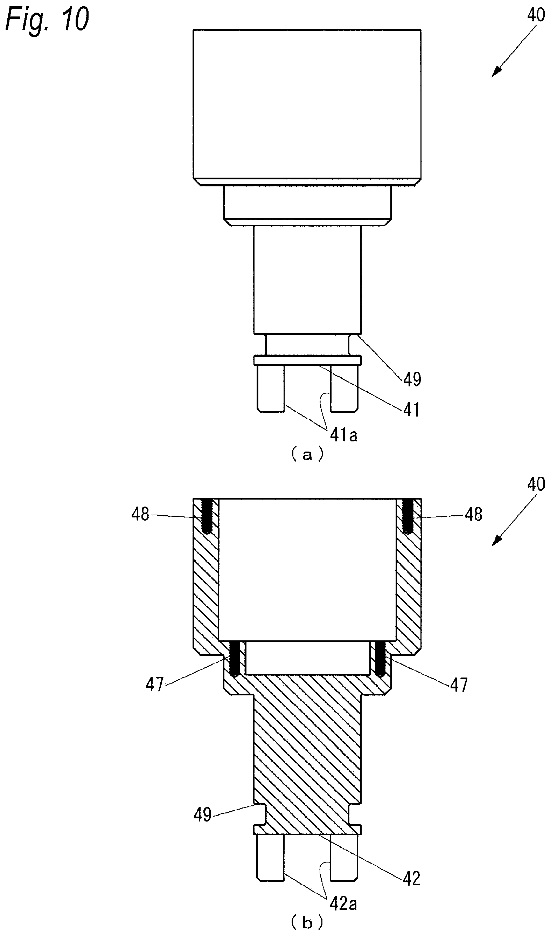

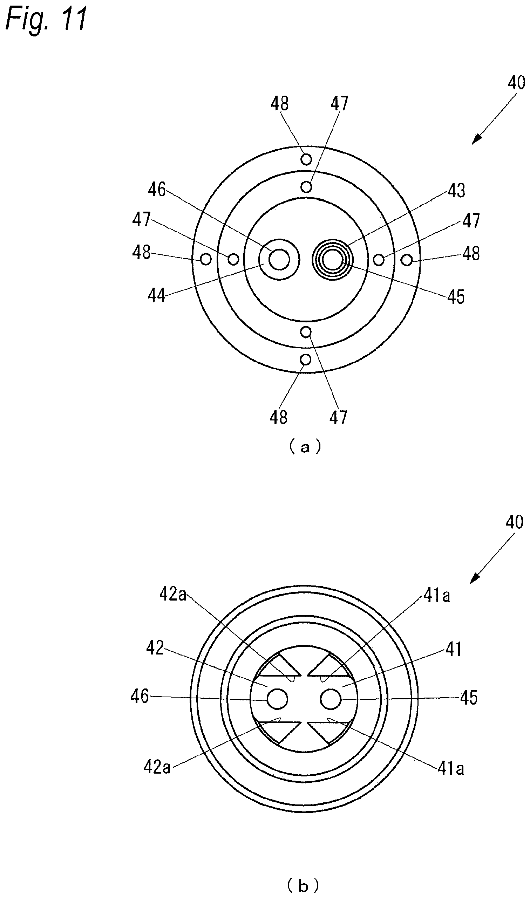

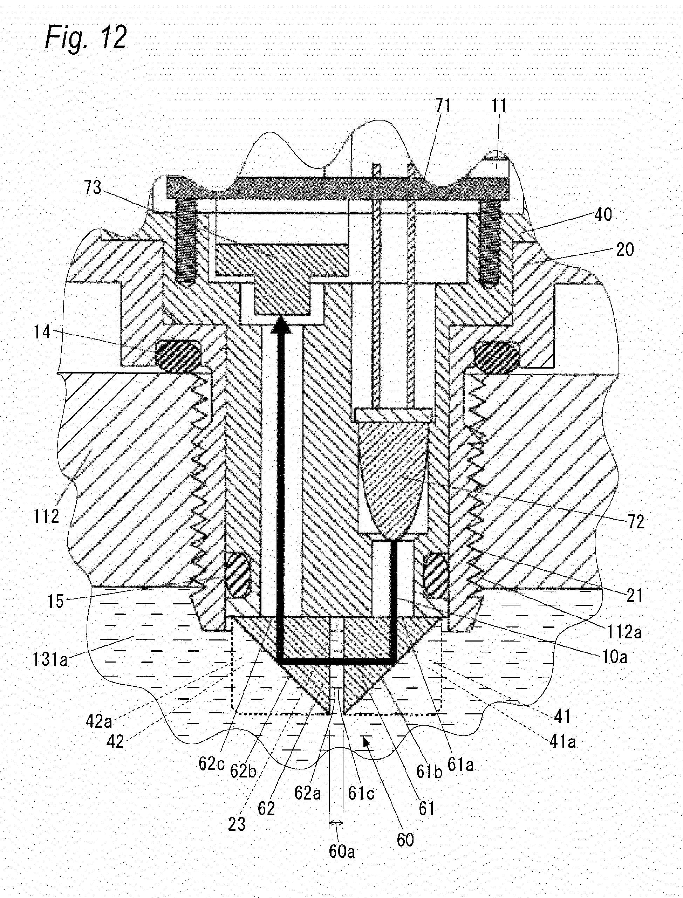

Fig. 9(a) is a front view of the holder 40. Fig. 9(b) is a front sectional view of the holder 40. Fig. 10(a) is a side view of the holder 40. Fig. 10(b) is a side sectional view of the holder 40. Fig. 11(a) is a plan view of the holder 40. Fig. 11(b) is a bottom view of the holder 40. Fig. 12 is a diagram showing an optical path 10a from the white LED 72 to the RGB sensor 73.

As shown in Figs.3 to 5(b) and Figs. 9(a) to 12, the holder 40 includes a prism housing portion 41 for housing the right-angle prism 61, a prism housing portion 42 for housing the right-angle prism 62, an LED housing portion 43 for housing the white LED 72, and an RGB sensor housing portion 44 for housing the RGB sensor 73. Further, the holder 40 is provided with a hole 45 penetrating the prism housing portion 41 and the LED housing portion 43, a hole 46 penetrating the prism housing portion 42 and the RGB sensor housing portion 44, screw holes 47 in which the screws 11 are threaded, screw holes 48 in which the bolts 13 with hexagon holes are threaded, and a groove 49 in which the O ring 15 is fitted.

The prism housing portion 41 includes two walls 41a sandwiching the right-angle prism 61 therebetween. The right-angle prism 61 is fixed to the walls 41a by means of adhesive. The prism housing portion 42 includes two walls 42a sandwiching the right-angle prism 62 therebetween. The right-angle prism 62 is fixed to the walls 42a by means of adhesive.

The holder 40 surrounds at least a part of the optical path 10a from the white LED 72 to the RGB sensor 73 by means of the LED housing portion 43, the hole 45, the prism housing portion 41, the prism housing portion 42, the hole 46 and the RGB sensor housing portion 44, thereby constituting an optical path surrounding portion according to this invention.

The surface of the holder 40 is treated to a treatment for preventing light reflection such as a black alumite treatment for matting.

The holder 40 supports the white LED 72 and the RGB sensor 73 via the circuit board 71. Further, the holder 40 directly supports the gap forming member 60.

As shown in Fig. 12, the oil gap 60a of the gap forming member 60 is disposed on the optical path 10a from the white LED 72 to the RGB sensor 73.

The right-angle prisms 61, 62 transmit light emitted from the white LED 72. The right-angle prism 61 is provided with a light incident surface 61a on which light emitted from the white LED 72 is made incident, a light reflection surface 61b which reflects the light entered from the light incident surface 61a in a manner of bending the propagation direction of the light by 90 degrees, and a light emission surface 61c which emits the light reflected by the light reflection surface 61b. The right-angle prism 62 is provided with a light incident surface 62a on which light emitted from the light emission surface 61c of the right-angle prism 61 is made incident, a light reflection surface 62b which reflects the light entered from the light incident surface 62a in a manner of bending the propagation direction of the light by 90 degrees, and a light emission surface 62c which emits the light reflected by the light reflection surface 62b.

The light emission surface 61c of the right-angle prism 61 and the light incident surface 62a of the right-angle prism 62 constitute gap forming surfaces as surfaces forming the oil gap 60a.

Each of the light incident surface 61a, the light reflection surface 61b and the light emission surface 61c of the right-angle prism 61 and the light incident surface 62a, the light reflection surface 62b and the light emission surface 62c of the right-angle prism 62 is optically polished. Further, each of the light reflection surface 61b of the right-angle prism 61 and the light reflection surface 62b of the right-angle prism 62 is provided with an aluminum deposition film. Furthermore, an SiO2 film is formed on the aluminum deposition film in order to protect the aluminum deposition film which is low in a degree of hardness and an adhesive force.

The optical path 10a is bent by 90 degrees by the light reflection surface 61b of the right-angle prism 61 and also bent by 90 degrees by the light reflection surface 62b of the right-angle prism 62. That is, the optical path 10a is bent by 180 degrees by the gap forming member 60.

When the distance of the oil gap 60a is too short, since the contaminant within the lubricant 131a unlikely flows suitably through the gap 60a, the detection accuracy of the colors of the contaminant within the lubricant 131a degrades. On the other hand, when the distance of the oil gap 60a is too long, the light emitted from the white LED 72 is excessively absorbed by the contaminant within the lubricant 131a within the oil gap 60a and hence unlikely reaches the RGB sensor 73. Thus, the detection accuracy of the colors of the contaminant within the lubricant 131a also degrades. Accordingly, preferably, the distance of the oil gap 60a is set suitably so that the detection accuracy of the colors of the contaminant within the lubricant 131a becomes high. A distance between the light emission surface 61c of the right-angle prism 61 and the light incident surface 62a of the right-angle prism 62, that is, the distance of the oil gap 60a is 1mm, for example.

The white LED 72 is an electronic component which emits white light and constitutes a light emitting element according to this invention. As the white LED 72, NSPW500GS-K1 manufactured by Nichia Corporation, for example, may be employed.

The RGB sensor 73 is an electronic component which detects the colors of the received light and constitutes a color light reception element according to this invention. As the RGB sensor 73, S9032-02 manufactured by Hamamatsu Photonics K.K., for example, may be employed.

As shown in Fig. 4, the connector 78 is configured in a manner that the connector 95 of the external device at the outside of the lubricant deterioration sensor 139 is connected thereto so as to be supplied with electric power via the connector 95 from the external device. Further, the connector 78 is configured to output the detection result of the lubricant deterioration sensor 139 as an electric signal to the external device via the connector 95.

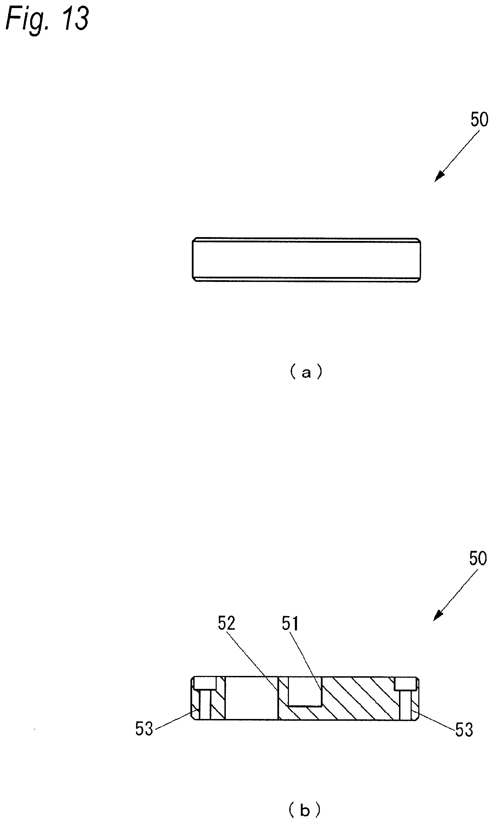

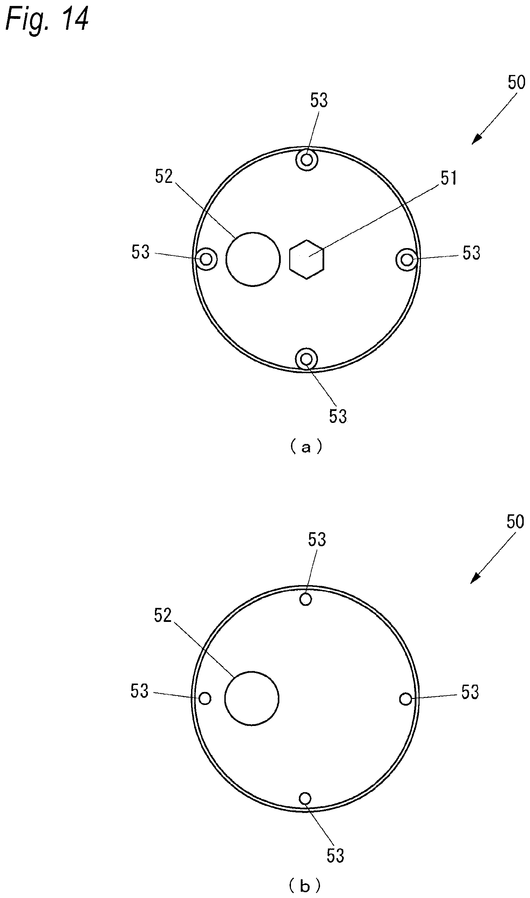

Fig. 13(a) is a front view of the holder cap 50. Fig. 13(b) is a front sectional view of the holder cap 50. Fig. 14(a) is a plan view of the holder cap 50. Fig. 14(b) is a bottom view of the holder cap 50.

As shown in Figs. 3 to 5(b) and Figs. 13(a) to 14(b), the holder cap 50 is provided with a tool contact portion 51 for contacting with a tool such as a hexagonal wrench at the time of rotating the supporting member 30 with respect to the housing 20. The tool contact portion 51 is a portion for receiving a rotational driving force of the supporting member 30 with respect to the housing 20 from the outside by a contact force, and constitutes a rotational driving force receiving portion according to this invention. The tool contact portion 51 is disposed at a position not contacting with the lubricant 131a when the housing 20 is fixed to the arm 112. Further, the holder cap 50 is provided with a hole 52 in which the connector 78 is inserted and holes 53 in which the bolts 13 with hexagon holes are inserted.

The holder cap 50 has a surface which is treated to the treatment for preventing light reflection such as the black alumite treatment for matting.

As shown in Figs. 3 and 4, the bolts 12 with hexagon holes are configured so as to prevent the rotation of the supporting member 30 with respect to the housing 20 by contacting with both the supporting member 30 and the housing 20, and constitute a rotation preventing member according to this invention. The bolt 12 with a hexagon hole has a tool contact portion 12a for contacting with a tool such as the hexagonal wrench. The tool contact portion 12a is a portion for receiving a driving force for contacting with both the supporting member 30 and the housing 20 from the outside by a contact force, and constitutes a contact driving force receiving portion according to this invention. The tool contact portion 12a is disposed at a position not contacting with the lubricant 131a when the housing 20 is fixed to the arm 112.

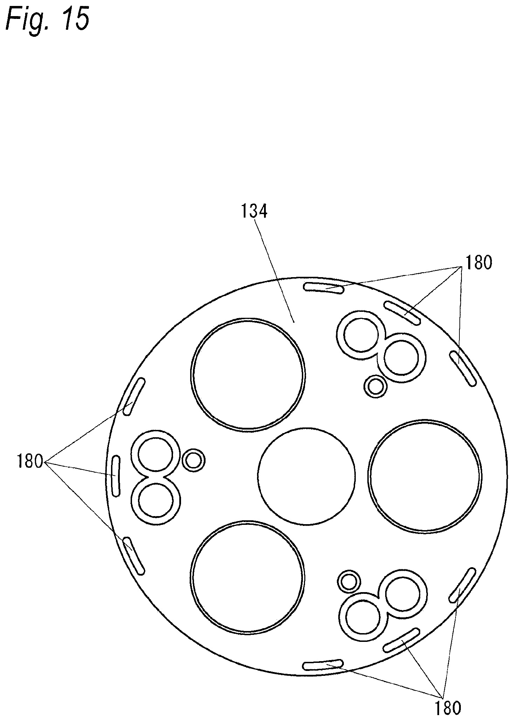

Fig. 15 is a plan view of a component of the supporting body 134 to which cleaning members 180 are fixed.

As shown in Fig. 2 and Fig. 15, each of the cleaning members 180 is disposed at a position contacting with both the light emission surface 61c of the right-angle prism 61 and the light incident surface 62a of the right-angle prism 62 in a case where the arm 112 and the supporting body 134 move relatively. In this respect, this arm acts as a sensor side portion of this invention, at which the lubricant deterioration sensor 139 is mounted, and the supporting body acts as a cleaning member side portion of this invention, at which the cleaning members 180 are mounted. The cleaning members 180 are disposed in a manner that three sets each configured by the three cleaning members are disposed around the center axis of the reducer 131 with a constant interval therebetween. Thus, the nine cleaning members 180 in total are disposed around the center axis of the reducer 131.

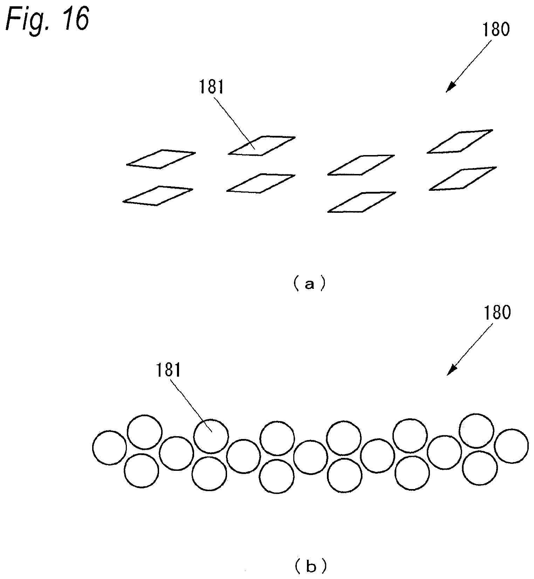

Fig. 16(a) is a plan view of an example of the cleaning member 180. Fig. 16(b) is a plan view of another example of the cleaning member 180 different from that shown in Fig. 16(a).

As shown in Figs. 16(a) and 16(b), various configurations can be employed as the shape of the cleaning member 180. Each of the cleaning members 180 has a plurality of insertion portions 181 each of which has elasticity and is inserted into the oil gap 60a of the lubricant deterioration sensor 139.

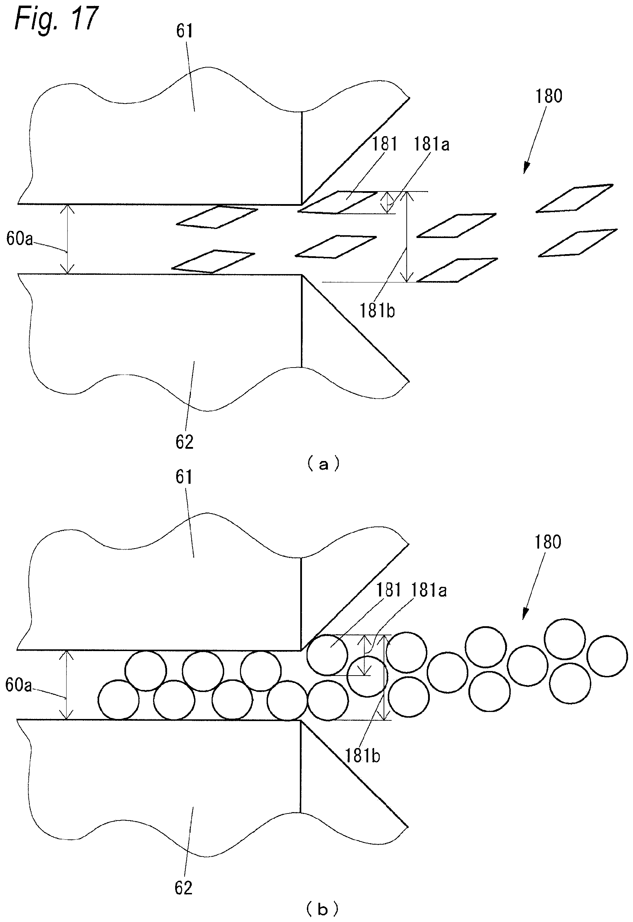

Fig. 17(a) is a plan view of the cleaning member 180 in a case where a part of the cleaning member 180 shown in Fig. 16(a) is inserted in the oil gap 60a. Fig. 17(b) is a plan view of the cleaning member 180 in a case where a part of the cleaning member 180 shown in Fig. 16(b) is inserted in the oil gap 60a.

As shown in Figs. 17(a) and 17(b), the width 181a of each of the insertion portions 181 in the distance direction of the oil gap 60a of the lubricant deterioration sensor 139 is smaller than the distance of the oil gap 60a. Further, the entire width 181b of the plurality of insertion portions 181 in the distance direction of the oil gap 60a is larger than the distance of the oil gap 60a.

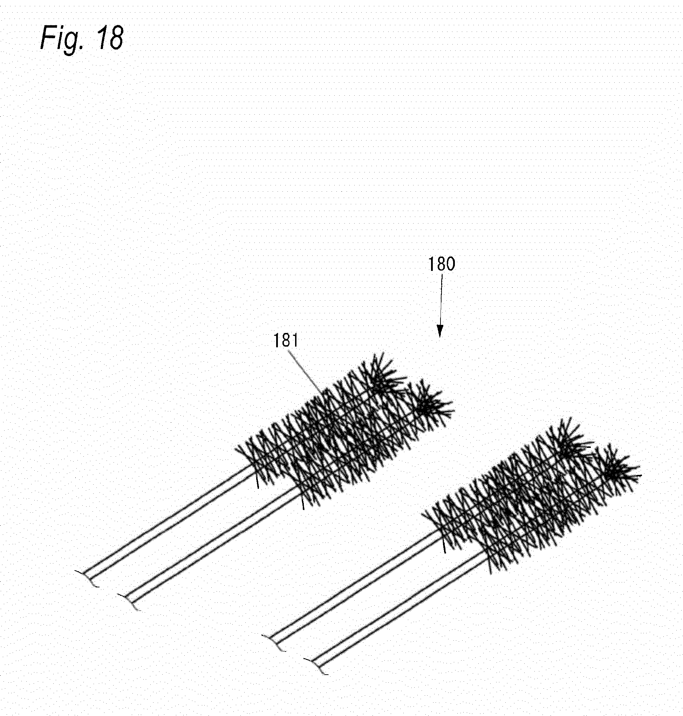

Fig. 18 is a perspective view of the cleaning member 180 in a case that the insertion portion 181 is a brush.

The insertion portion 181 may be a wiper made of rubber, for example, or a brush as shown in Fig. 18.

Although the explanation is made above as to the cleaning member 180, the configuration of the cleaning member 190 is substantially same. Each of the cleaning members 190 is disposed at a position contacting with the gap forming surfaces of the lubricant deterioration sensor 137 in a case where the supporting body 134 and the crank shaft 135b move relatively. In this respect, the supporting body acts as the sensor side portion of this invention at which the lubricant deterioration sensor 137 is mounted, and the crank shaft acts as the cleaning member side portion of this invention at which the cleaning members 190 are mounted.

Next, the assembling method of the lubricant deterioration sensor 139 will be explained. Although the explanation is made hereinafter as to the lubricant deterioration sensor 139, the configuration of each of the lubricant deterioration sensors such as the lubricant deterioration sensor 137 other than the lubricant deterioration sensor 139 is substantially same.

First, adhesive is pasted on the surface plane of the prism housing portion 41 of the holder 40 to be made in contact with the light incident surface 61a of the right-angle prism 61, and also adhesive is pasted on the two surface planes of the right-angle prism 61 to be made in contact with the two walls 41a of the prism housing portion 41. Then, the right-angle prism 61 is fixed to the prism housing portion 41 by the adhesive. Further, adhesive is pasted on the surface plane of the prism housing portion 42 of the holder 40 to be made in contact with the light emission surface 62c of the right-angle prism 62, and also adhesive is pasted on the two surface planes of the right-angle prism 62 to be made in contact with the two walls 42a of the prism housing portion 42. Then, the right-angle prism 62 is fixed to the prism housing portion 42 by the adhesive. Furthermore, the white LED 72 is fixed to the LED housing portion 43 of the holder 40 by means of adhesive.

Then, the circuit board 71 mounting the RGB sensor 73 thereon is fixed to the holder 40 by means of the screws 11, and the white LED 72 is fixed to the circuit board 71 by means of soldering. Further, various kinds of electronic components such as the connector 78 are assembled, thereby supporting the electronic component group 70 by the holder 40.

Next, the holder cap 50 is fixed to the holder 40 by means of the bolts 13 with hexagon holes.

Lastly, the holder 40 attached with the O ring 15 is fixed, by means of the bolts 12 with hexagon holes, to the holder housing portion 23 of the housing 20 attached with the O ring 14 and the O ring 16.

Next, the explanation will be made as to a method of mounting the lubricant deterioration sensor 139 to the arm 112. Although the explanation is made hereinafter as to the lubricant deterioration sensor 139, the configuration of each of the lubricant deterioration sensors such as the lubricant deterioration sensor 137 other than the lubricant deterioration sensor 139 is substantially same.

First, the tool contact portion 22 of the housing 20 is grasped by a tool and the screw portion 21 of the housing 20 is threaded into the screw hole 112a of the arm 112, thereby fixing the lubricant deterioration sensor 139 to the arm 112.

Then, the connector 95 of the external device at the outside of the lubricant deterioration sensor 139 is connected to the connector 78.

Next, the operation of the industrial robot 100 will be explained.

First, the operation of the articular portion 130 will be explained. Although the explanation is made hereinafter as to the articular portion 130, the configuration of each of the articular portions 120, 140 to 170 is substantially same.

When the output shaft of the motor 138 of the articular portion 130 rotates, the rotation force of the motor 138 is reduced by the reducer 131, to thereby move the arm 113 fixed to the supporting body 134 of the recorder 131 with respect to the arm 112 fixed to the case 133 of the reducer 131.

In this case, in accordance with the relative movement between the arm 112 and the supporting body 134 of the recorder 131, the cleaning member 180 provided at the supporting body 134 is inserted in the oil gap 60a of the lubricant deterioration sensor 139 provided at the arm 112 as shown in Fig. 17. Then, by the cleaning member 180 inserted in the oil gap 60a of the lubricant deterioration sensor 139, dirt such as sludge adhered to the gap forming surfaces of the lubricant deterioration sensor 139, that is, the light emission surface 61c of the right-angle prism 61 and the light incident surface 62a of the right-angle prism 62 is rubbed off.

Similarly, in accordance with the relative movement between the supporting body 134 and the crank shaft 135b of the recorder 131, the cleaning member 190 provided at the crank shaft 135b is inserted in the oil gap of the lubricant deterioration sensor 137 provided at the supporting body 134. Then, by the cleaning member 190 inserted in the oil gap of the lubricant deterioration sensor 137, dirt such as sludge adhered to the gap forming surfaces of the lubricant deterioration sensor 137 is rubbed off.

Next, the operation of the lubricant deterioration sensor 139 will be explained. Although the explanation is made hereinafter as to the lubricant deterioration sensor 139, the configuration of each of the lubricant deterioration sensors such as the lubricant deterioration sensor 137 other than the lubricant deterioration sensor 139 is substantially same.

The lubricant deterioration sensor 139 emits white light from the white LED 72 in response to electric power supplied from the external device via the connector 78.

Then, the lubricant deterioration sensor 139 outputs light amounts of respective colors RGB of the light received by the RGB sensor 73 as an electric signal to the external device via the connector 78.

The lubricant deterioration sensor 139 may additionally mount another sensor other than the RGB sensor 73. For example, in the lubricant deterioration sensor 139, when a temperature sensor for detecting the temperature of the lubricant 131a is contained in the electronic component group 70, the temperature detected by the temperature sensor can also be outputted as an electric signal to the external device via the connector 78.

Next, the explanation will be made as to a method of adjusting the direction of the opening 60b of the oil gap 60a of the lubricant deterioration sensor 139 (see Fig. 5(b)). Although the explanation is made hereinafter as to the lubricant deterioration sensor 139, the configuration of each of the lubricant deterioration sensors such as the lubricant deterioration sensor 137 other than the lubricant deterioration sensor 139 is substantially same.

First, each of the bolts 12 with hexagon holes is loosened by a tool inserted into the tool contact portion 12a so that the supporting member 30 becomes rotatable with respect to the housing 20.

Then, in a state that the rotation of the housing 20 with respect to the arm 112 is prevented by grasping the tool contact portion 22 of the housing 20 by the tool, the supporting member 30 is rotated with respect to the housing 20 by the tool inserted into the tool contact portion 51. The direction of the opening 60b of the oil gap 60a changes in accordance with the rotation of the supporting member 30 with respect to the housing 20.

Lastly, each of the bolts 12 with hexagon holes is fastened by the tool inserted into the tool contact portion 12a so that the rotation of the supporting member 30 becomes impossible with respect to the housing 20.

As explained above, the RGB sensor 73 detects colors with respect to the light having wavelengths not absorbed by the contaminant within the lubricant 131a at the oil gap 60a among the white light emitted by the white LED 72. Thus, each of the lubricant deterioration sensors such as the lubricant deterioration sensor 139 can immediately detect the colors of the contaminant within the lubricant 131a of the reducer 131. That is, each of the lubricant deterioration sensors enables to immediately specify the kinds and amounts of the contaminant within the lubricant 131a of the reducer 131 based on the colors detected by the RGB sensor 73, by using the external device such as a computer. Each of the lubricant deterioration sensors may be configured that the electronic component group 70 contains an electronic component which specifies the kinds and amounts of the contaminant within the lubricant based on the colors detected by the RGB sensor 73.

Generally, in the industrial robot, accuracy of the track of the arm etc. largely depends on the performance of the reducer used at the articular portion. Thus, it is important to suitably exchange the reducer for the industrial robot for new one when the performance of the reducer degrades. However, in the case of exchanging the reducer for the industrial robot, it is required to stop the industrial robot provided with this reducer and a production line mounting the industrial robot. Thus, in order to grasp the exchange time of the reducer for the industrial robot, it is very important to suitably predict a failure of the reducer for the industrial robot. In this respect, as described above, each of the lubricant deterioration sensors of the industrial robot 100 enables to immediately specify the kinds and amounts of the contaminant within the lubricant 131a of the reducer 131 based on the colors detected by the RGB sensor 73, by using the external device such as a computer. Thus, the industrial robot 100 and each of the reducers of the industrial robot 100 enables to perform immediate prediction of a failure.

To the lubricant 131a, there is sometimes added various kinds of additive such as friction reducing agent like organic molybdenum such as MoDTC or MoDTP for reducing the friction of a friction surface, extreme-pressure additive such as SP-based additive for improving extreme-pressure lubricity representing the performance for suppressing the sticking of the friction surface, and dispersing agent such as calcium sulfonate for suppressing the generation and adhesion of sludge. These additives are separated from the lubricant 131a in accordance with the deterioration of the lubricant 131a in such a manner that the additive adheres to, binds with or settles on the metal surface of the industrial robot 100 and the reducer. Each of the lubricant deterioration sensors can specify, based on the detected colors, not only an amount of ion powder within the lubricant 131a but also a deterioration degree of the base oil and increase of the contaminant such as sludge due to the reduction of various kinds of additive added to the lubricant 131a. Thus, each of the industrial robot 100 and the reducers of the industrial robot 100 can improve the prediction accuracy of a failure as compared with a technique where a failure of the reducer is predicted only based on a density of iron powder.

Thus, the inventors of the present application have analyzed the sludge adhered to the gap forming member in order to ascertain the cause of the generation of the sludge. As a result, the inventors of the present application have ascertained that molybdenum (Mo) is contained in the sludge adhered to the gap forming member.

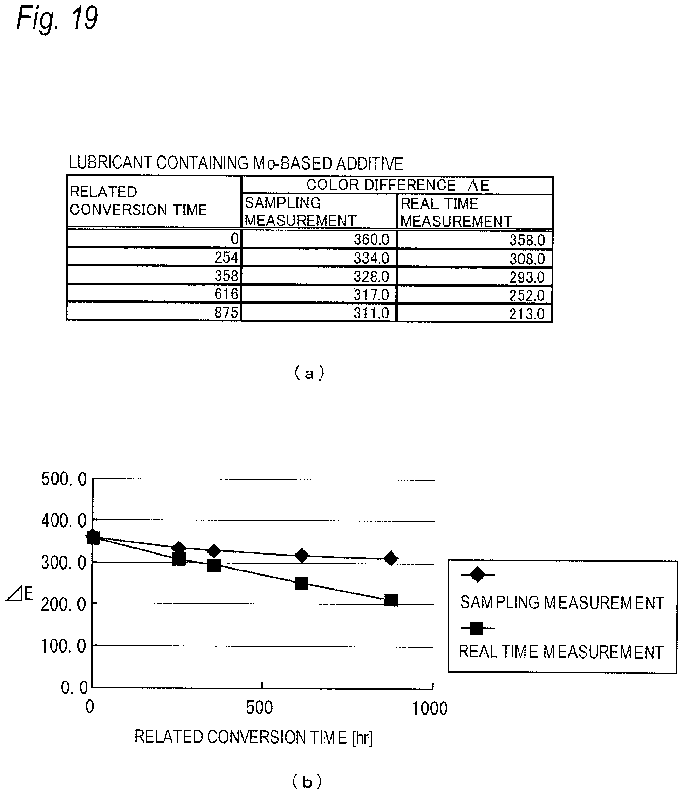

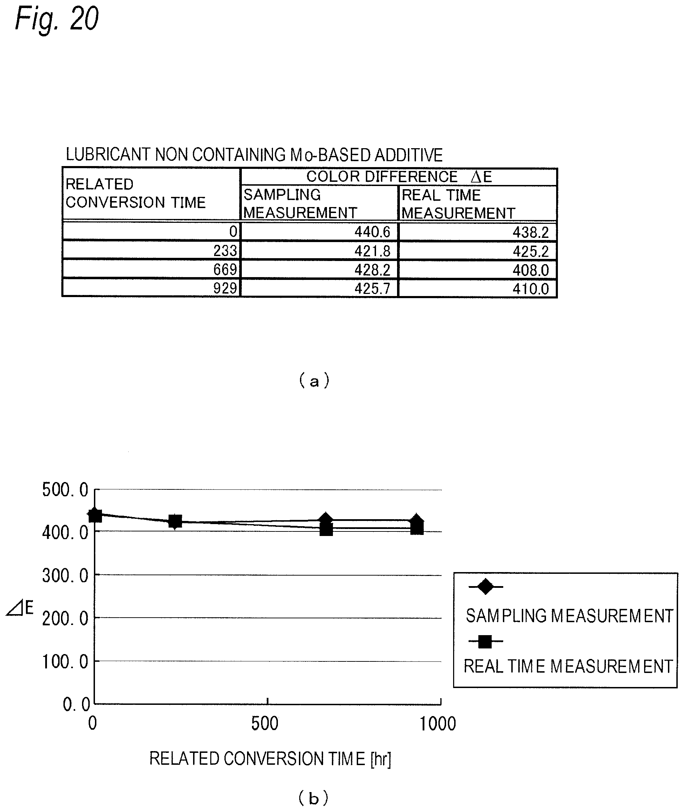

Then, in order to investigate whether or not molybdenum contained in the lubricant 131a as the additive is a cause of the degradation of the performance of the lubricant deterioration sensor, the inventors of the present application have performed the comparative experimentation as to the degradation of the performance of the lubricant deterioration sensor between the lubricant 131a containing molybdenum as the additive and the lubricant 131a not containing molybdenum as the additive. In a device for the experimentation prepared so as to have the substantially same configuration as the articular portion 130, under a condition that the maximum output rotation speed was 15 rpm, the maximum load torque was 2.5 times as large as the rated torque of the reducer 131 and a load moment was the rated moment of the reducer 131, this experimentation was performed in a following manner. That is, a reciprocal rotation movement of rotating the supporting body 134 by 45 degrees in a reverse direction with respect to the case 133 after rotating the supporting body by 45 degrees in a forward direction with respect to the case was repeatedly continued. In this device for the experimentation, the configuration not provided with the cleaning member according to this invention is employed so that the sludge adhered to the gap forming member cannot be rubbed off. The lubricant used in this experimentation as the lubricant 131a containing molybdenum as the additive contains organic molybdenum as the friction reducing agent, the extreme-pressure additive, the dispersing agent and antioxidant each in a rage of 0.1 % to 10% as the additive. As compared with the lubricant used in this experimentation as the lubricant 131a containing molybdenum as the additive, the lubricant used in this experimentation as the lubricant 131a not containing molybdenum as the additive differs only in a point that the organic molybdenum as the friction reducing agent is not contained. The results of this experimentation are shown in Figs. 19 and 20.

Fig. 19(a) is a table showing the experimentation result of the chronological change of the color difference ΔE in a case where the lubricant 131a contains molybdenum as the additive. Fig. 19(b) is a graph of the experimentation result shown in Fig. 19(a). Fig. 20(a) is a table showing the experimentation result of the chronological change of the color difference ΔE in a case where the lubricant 131a does not contain molybdenum as the additive. Fig. 20(b) is a graph of the experimentation result shown in Fig. 20(a).

The color difference ΔE represents a color difference ΔE of the colors detected by the RGB sensor 73 with respect to black. The color difference ΔE of the colors detected by the RGB sensor 73 with respect to black can be calculated by an expression shown by the following Math. 1 by using the respective values of the colors RGB detected by the RGB sensor 73.

[Math. 1]

$$\Delta E = \sqrt{R^2 + G^2 + B^2}$$

In Figs. 19 and 20, a rated conversion time was obtained in a manner that a time period during which the device for the experimentation was actually driven was converted into a time period of a case that the output rotation speed is 15 rpm and the load torque is the rated torque of the reducer 131, based on the output rotation speed and the load torque of the reducer 131 in a case where the device for the experimentation was actually driven. The life time of the reducer 131 is defined as 6,000 hours in a case where the reducer 131 is continuously driven under a condition that the output rotation speed is 15 rpm and the load torque is the rated torque of the reducer 131. Further, the sampling measurement was performed in a manner that the lubricant 131a was drained from the device for the experimentation each time of the measurement, and a color difference ΔE of the colors of the lubricant 131a drained from the device for the experimentation with respect to black was measured by using a lubricant deterioration sensor like the lubricant deterioration sensor 139. Further, the real time measurement was performed in a manner that the color difference ΔE of the colors of the lubricant 131a within the device for the experimentation with respect to black was measured by using the lubricant deterioration sensor 139 attached to the device for the experimentation.

Since the right-angle prisms of the gap forming member of the lubricant deterioration sensor used at the time of the sampling measurement are made in contact with the lubricant 131a only at the time of the measurement, sludge generated by the deterioration of the lubricant 131a does not adhere to the prisms. Thus, the experimentation result of the sampling measurement represents the state of the lubricant 131a quite precisely.

On the other hand, since the right-angle prisms 61, 62 of the gap forming member 60 of the lubricant deterioration sensor 139 used at the time of the real time measurement are always made in contact with the lubricant 131a, sludge generated by the deterioration of the lubricant 131a adheres to the prisms. Thus, the experimentation result of the real time measurement is influenced by the sludge adhered to the right-angle prisms 61, 62.

Therefore, a difference between the experimentation result of the sampling measurement and the experimentation result of the real time measurement represents a degree of the influence of the sludge adhered to the right-angle prisms 61, 62.

As shown in Figs. 19 and 20, as compared with the case where the lubricant 131a contains molybdenum as the additive, when the lubricant 131a does not contain molybdenum as the additive, the difference between the experimentation result of the sampling measurement and the experimentation result of the real time measurement is small even if the experimentation time periods are substantially same therebetween. In other words, it is clear that this case is less influenced by the sludge adhered to the right-angle prisms 61, 62. As a result, the inventors of the present application have concluded that molybdenum contained in the lubricant as the additive is at least one cause of the degradation of the performance of the lubricant deterioration sensor 139.

As explained above, when the lubricant 131a does not contain molybdenum as the additive, each of the industrial robot 100 and the reducers of the industrial robot 100 can prevent a phenomenon that molybdenum adheres to the right-angle prisms of the gap forming member as the sludge. Thus, the adhesion of the sludge to the right-angle prisms of the gap forming member can be suppressed and hence the degradation of the performance of the lubricant deterioration sensor can be suppressed. As a result, each of the industrial robot 100 and the reducers of the industrial robot 100 can suppress the degradation of the performance of the lubricant deterioration sensor which enables to immediately specify the kinds and amounts of the contaminant within the lubricant 131a.

In the industrial robot 100, each of the cleaning members 180 is disposed at a position contacting with the gap forming surfaces of the lubricant deterioration sensor 139, that is, both the light emission surface 61c of the right-angle prism 61 and the light incident surface 62a of the right-angle prism 62 in a case where the reducer 131 rotates. Thus, the cleaning member 180 can rub off dirt such as sludge adhered to the gap forming surfaces of the lubricant deterioration sensor 139 each time the arm 112 is driven by the reducer 131. In the industrial robot 100, when the arm 112 as the sensor side portion and the supporting body 134 as the cleaning member side portion move relatively, the cleaning member 180 rubs off dirt such as sludge adhered to the gap forming surfaces of the gap forming member 60 of the lubricant deterioration sensor 139. Thus, the degradation of the performance of the lubricant deterioration sensor 139 can be suppressed. Therefore, the industrial robot 100 can suppress the degradation of the performance of the lubricant deterioration sensor 139 which enables to immediately specify the kinds and amounts of contaminant within the lubricant 131a.

In the similar manner, in each of the industrial robot 100 and the reducers of the industrial robot 100, each of the cleaning members 190 is disposed at a position contacting with the gap forming surfaces of the lubricant deterioration sensor 137 in a case where the reducer 131 rotates. Thus, the cleaning member 190 can rub off dirt such as sludge adhered to the gap forming surfaces of the lubricant deterioration sensor 137 each time the reducer 131 rotates. In each of the industrial robot 100 and the reducers of the industrial robot 100, in a case where the supporting body 134 as the sensor side portion and the crank shaft 135b as the cleaning member side portion move relatively, the cleaning member 190 rubs off dirt such as sludge adhered to the gap forming surfaces of the lubricant deterioration sensor 137. Thus, the degradation of performance of the lubricant deterioration sensor 137 can be suppressed. Accordingly, each of the industrial robot 100 and the reducers of the industrial robot 100 can suppress the degradation of performance of the lubricant deterioration sensor 137 which enables to immediately specify the kinds and amounts of contaminant within the lubricant 131a.

Further, as described above, the industrial robot 100 can suppress the degradation of performance of the lubricant deterioration sensors 137 and 139 each of which enables to immediately specify the kinds and amounts of contaminant within the lubricant 131a. Thus, the accuracy of immediate prediction of a failure can be maintained for a long time.

Further, as described above, the reducer for an industrial robot, that is, each of the reducers for the industrial robot 100 can suppress the degradation of performance of the lubricant deterioration sensors 137 and 139 each of which enables to immediately specify the kinds and amounts of contaminant within the lubricant 131a. Thus, the accuracy of immediate prediction of a failure can be maintained for a long time.

As described above, the width 181a of each of the insertion portions 181 of the cleaning member 180 in the distance direction of the oil gap 60a of the lubricant deterioration sensor 139 is smaller than the distance of the oil gap 60a. Further, the entire width 181b of the plurality of insertion portions 181 of the cleaning member 180 in the distance direction of the oil gap 60a is larger than the distance of the oil gap 60a. Thus, in the industrial robot 100, as compared with a case where the cleaning member 180 is configured only by a single insertion portion which width in the distance direction of the oil gap 60a is larger than the distance of the oil gap 60a, the pressure applied to the lubricant deterioration sensor 139 by the cleaning member 180 at the time of inserting the cleaning member 180 in the oil gap 60a can be mad small. As a result, the degradation of performance of the lubricant deterioration sensor 139 due to the pressure applied to the lubricant deterioration sensor 139 from the cleaning member 180 can be suppressed. Further, in the industrial robot 100, dirt such as sludge adhered to the gap forming surfaces of the gap forming member 60 of the lubricant deterioration sensor 139, that is, both the light emission surface 61c of the right-angle prism 61 and the light incident surface 62a of the right-angle prism 62 can be rubbed off by the plurality of insertion portions 181 of the cleaning member 180.

Similarly, the width of each of the insertion portions of the cleaning member 190 in the distance direction of the oil gap of the lubricant deterioration sensor 137 is smaller than the distance of the oil gap of the lubricant deterioration sensor 137. Further, the entire width of the plurality of insertion portions of the cleaning member 190 in the distance direction of the oil gap of the lubricant deterioration sensor 137 is larger than the distance of the oil gap of the lubricant deterioration sensor 137. Thus, in each of the industrial robot 100 and the reducers of the industrial robot 100, as compared with a case where the cleaning member 190 is configured only by a single insertion portion which width in the distance direction of the oil gap of the lubricant deterioration sensor 137 is larger than the distance of the oil gap of the lubricant deterioration sensor 137, the pressure applied to the lubricant deterioration sensor 137 by the cleaning member 190 at the time of inserting the cleaning member 190 in the oil gap of the lubricant deterioration sensor 137 can be mad small. As a result, the degradation of performance of the lubricant deterioration sensor 137 due to the pressure applied to the lubricant deterioration sensor 137 from the cleaning member 190 can be suppressed. Further, in each of the industrial robot 100 and the reducers of the industrial robot 100, dirt such as sludge adhered to the gap forming surfaces of the gap forming member of the lubricant deterioration sensor 137 can be effectively rubbed off by the plurality of insertion portions of the cleaning member 190.

The lubricant deterioration sensor 139 is provided at the outside thereof with the cleaning members 180 for rubbing off dirt such as sludge adhered to the gap forming surfaces. Thus, the configuration of this sensor can be miniaturized as compared with a case where the lubricant deterioration sensor itself is provided with a configuration for rubbing off dirt such as sludge adhered to the gap forming surfaces. Although the explanation is made as to the lubricant deterioration sensor 139, the aforesaid explanation is substantially applied to the lubricant deterioration sensor 137.

In each of the lubricant deterioration sensors such as the lubricant deterioration sensor 139, the housing 20 supports the supporting member 30 so as to be rotatable in a manner that the direction of the opening 60b of the oil gap 60a changes when the supporting member 30 rotates. Thus, the direction of the opening 60b of the oil gap 60a in the case of fixing the housing 20 to the industrial robot 100 can be adjusted so that the dirt cleaning effects of the cleaning members 180, 190 in the case of fixing the housing 20 to the industrial robot 100 can be enhanced. As a result, each of the industrial robot 100 and the reducers of the industrial robot 100 enables to predict a failure with a high accuracy.

Further, in each of the lubricant deterioration sensors such as the lubricant deterioration sensor 139, the supporting member 30 includes the tool contact portion 51 at a position not contacting with the lubricant 131a when the housing 20 is fixed to the industrial robot 100. This tool contact portion acts as the portion for receiving the rotational driving force with respect to the housing 20 from the outside by the contact force. Thus, in each of the lubricant deterioration sensors, the direction of the opening 60b of the oil gap 60a in the case of fixing the housing 20 to the industrial robot 100 can be adjusted so that the dirt cleaning effects of the cleaning members 180, 190 can be enhanced after fixing the housing 20 to the industrial robot 100.

Furthermore, in each of the lubricant deterioration sensors such as the lubricant deterioration sensor 139, each of the bolts 12 with hexagon holes includes the tool contact portion 12at the position not contacting with the lubricant 131a when the housing 20 is fixed to the industrial robot 100. In this respect, each of these bolts with hexagon holes is configured so as to prevent the rotation of the supporting member 30 with respect to the housing 20 by contacting with both the supporting member 30 and the housing 20. This tool contact portion acts as the portion for receiving the driving force for contacting with both the supporting member 30 and the housing 20 from the outside by the contact force. Thus, in each of the lubricant deterioration sensors, the direction of the opening 60b of the oil gap 60a in the case of fixing the housing 20 to the industrial robot 100 can be adjusted so that the dirt cleaning effects of the cleaning members 180, 190 can be enhanced after fixing the housing 20 to the industrial robot 100.

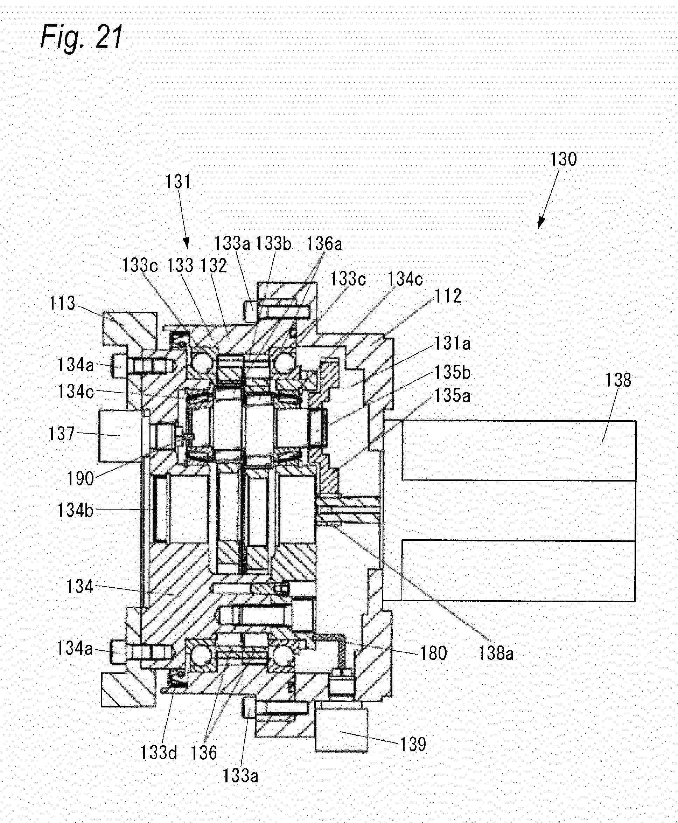

As shown in Fig. 2, the lubricant deterioration sensor 137 is disposed with respect to the cleaning member 190 in the extending direction of the rotation shaft of the cleaning member 190. Similarly, as shown in Fig. 2, the lubricant deterioration sensor 139 is disposed with respect to the cleaning member 180 in the extending direction of the rotation shaft of the cleaning member 180. However, the arrangement between the lubricant deterioration sensor and the cleaning member may not be limited thereto. For example, as shown in Fig. 21, the lubricant deterioration sensor 139 may be disposed with respect to the cleaning member 180 in a direction orthogonal to the extending direction of the rotation shaft of the cleaning member 180.

Furthermore, in each of the lubricant deterioration sensors, since the light emitting element is the white LED for emitting white light, the sensor can be miniaturized as compared with a configuration where the light emitting element is a lamp other than an LED, for example. Thus, each of the industrial robot 100 and the reducers of the industrial robot 100 can be miniaturized. The light emitting element according to this invention may be one other than the white LED. For example, the light emitting element may be a lamp other than an LED. Further, the light emitting element may be configured to include a red LED or a red lamp other than an LED, a green LED or a green lamp other than an LED and a blue LED or a blue lamp other than an LED, to thereby emit white light by composing light of respective colors emitted from these LEDs or these lamps other than LEDs.

Furthermore, in each of the lubricant deterioration sensors, the gap forming member 60 is provided with the light reflection surfaces 61b, 62b for bending the optical path 10a. Thus, as compared with the configuration where the optical path 10a from the white LED 72 to the RGB sensor 73 is straight, the entire configuration can be miniaturized by disposing the white LED 72 and the RGB sensor 73 close to each other. Furthermore, in each of the lubricant deterioration sensors, the gap forming member 60 has a function of bending the optical path 10a as well as a function of forming the oil gap 60a. Thus, the number of the components can be reduced as compared with a configuration where a member for bending the optical path 10a is separately provided in place of the gap forming member 60. As a result, each of the industrial robot 100 and the reducers of the industrial robot 100 can be miniaturized and also reduce the number of the components.

In particular, in each of the lubricant deterioration sensors, the gap forming member 60 is configured by the two right-angle prisms 61, 62 respectively provided with the light reflection surfaces 61b, 62b each for bending the optical path 10a by 90 degrees. Further, the optical path 10a is bent by 180 degrees by the light reflection surfaces 61b, 62b of the two right-angle prisms 61, 62, and the oil gap 60a is formed between the two right-angle prisms 61, 62. Thus, the sensor can be miniaturized with the simple configuration having a small number of the components. As a result, each of the industrial robot 100 and the reducers of the industrial robot 100 can be miniaturized with the simple configuration having the small number of the components.

Furthermore, each of the lubricant deterioration sensors is configured in a manner that the sensor has the holder 40 surrounding at least a part of the optical path 10a and the surface of the holder 40 is treated to the treatment for preventing light reflection. Thus, the RGB sensor 73 can be prevented from receiving unnecessary reflection light. As a result, as compared with a configuration where the RGB sensor 73 receives unnecessary reflection light, each of the lubricant deterioration sensors can improve the detection accuracy of the colors of the contaminant within the lubricant 131a. As a result, each of the industrial robot 100 and the reducers of the industrial robot 100 can improve the prediction accuracy of a failure.

Furthermore, in each of the lubricant deterioration sensors, an oil repelling treatment may be performed on the surface planes of the gap forming member 60 forming the oil gap 60a, that is, the light emission surface 61c of the right-angle prism 61 and the light incident surface 62a of the right-angle prism 62. In each of the lubricant deterioration sensors, when each of the light emission surface 61c of the right-angle prism 61 and the light incident surface 62a of the right-angle prism 62 is treated to the oil repelling treatment, the lubricant 131a flows through the oil gap 60a easily. Thus, as compared with a configuration where the lubricant 131a likely remains at the oil gap 60a, the detection accuracy of the colors of the contaminant within the lubricant 131a can be improved. Furthermore, in each of the lubricant deterioration sensors, when each of the light emission surface 61c of the right-angle prism 61 and the light incident surface 62a of the right-angle prism 62 is treated to the oil repelling treatment, dirt is unlikely adhered to each of the light emission surface 61c of the right-angle prism 61 and the light incident surface 62a of the right-angle prism 62. Thus, the degradation of the detection accuracy of the colors of the contaminant within the lubricant 131a due to the adhesion of dirt can be suppressed. As a result, each of the industrial robot 100 and the reducers of the industrial robot 100 can improve the prediction accuracy of a failure.

The prediction accuracy of a failure of the reducer can be improved by jointly using the lubricant deterioration sensor according to this invention, the temperature sensor for measuring the temperature of the lubricant and a monitoring mechanism for a current value of the motor etc.

Although each of the right-angle prisms 61, 62 of the gap forming member 60 is made of glass in this embodiment, each of them may be formed by material other than glass such as silicon resin. When each of the prisms 61, 62 is formed by silicon resin, dirt can be unlikely adhered to the surface planes thereof forming the oil gap 60a.

Although the gap forming member 60 is configured by the two right-angle prisms 61, 62 in this embodiment, the gap forming member may be configured by three or more prisms.

In each of the lubricant deterioration sensors, the white LED 72 and the RGB sensor 73 may be configured in an arrangement other than that explained in this embodiment. For example, in each of the lubricant deterioration sensors, the optical path 10a from the white LED 72 to the RGB sensor 73 may be straight

Further, in each of the lubricant deterioration sensors, the optical path 10a may be bent by employing a configuration other than the right-angle prisms.

In each of the lubricant deterioration sensors, for example, a battery such as a battery cell may be used as an electric power supply means. A wireless communication may be employed as a means for outputting the detection result to the external device.

Further, the mounting position of each of the lubricant deterioration sensors is not limited to that shown in this embodiment, and preferably may be set suitably according to the usage etc. of the industrial robot 100.

Although the machine according to this invention is the reducer for the industrial robot or the industrial robot in this embodiment, a machine other than them may be used.

Industrial Applicability

According to this invention, the machine is provided which can suppress the degradation of performance of the lubricant deterioration sensor that enables to immediately specify the kinds and amounts of the contaminant within the lubricant.

Reference Signs List

10a optical path
12 bolt with hexagon hole (rotation preventing member)
12a tool contact portion (contact driving force receiving portion)
20 housing (fixing member)
30 supporting member
40 holder (optical path surrounding portion)
51 tool contact portion (rotational driving force receiving portion)
60 gap forming member
60a oil gap
60b opening
61 right-angle prism
61b light reflection surface
61c light emission surface (gap forming surface)
62 right-angle prism
62a light incident surface (gap forming surface)
62b light reflection surface
72 white LED (light emitting element)
73 RGB sensor (color light reception element)
100 industrial robot (machine)
112 arm (sensor side portion)
113 - 116 arm

120, 130, 140, 150, 160, 170 articular portion
131 reducer (reducer for industrial robot, machine)
131a lubricant
132 reducer body (machine body)
134 supporting body (sensor side portion, cleaning member side portion)
135b crank shaft (cleaning member side portion)
137, 139 lubricant deterioration sensor
180 cleaning member
181 insertion portion
181a width (width of each of insertion portions)
181b width (entire width of a plurality of insertion portions)
190 cleaning member

**Claims**

1. A machine (131) configured to detect deterioration of lubricant, comprising:

   a machine body (132);
   a lubricant deterioration sensor (137, 139) which is configured to detect deterioration of the lubricant, which lubricant is for reducing friction generated at a movable portion of the machine body (132); and
   a cleaning member (180) which is configured to clean the lubricant deterioration sensor (137, 139),
   wherein
   the machine body (132) includes a sensor side portion at which the lubricant deterioration sensor (137, 139) is mounted and a cleaning member side portion which is movable with respect to the sensor side portion and at which the cleaning member (180) is mounted,
   the lubricant deterioration sensor includes a light emitting element (72) which is configured to emit light, a color light reception element (73) which is configured to detect color of received light, and a gap forming member (60) having gap forming surfaces (61c, 62a) at which an oil gap (60a) into which the lubricant (131a) enters is formed, the gap forming member (60) is configured to transmit the light emitted from the light emitting element (72),
   the oil gap (60a) is disposed on an optical path from the light emitting element (72) to the color light reception element (73), and
   the cleaning member (180) is disposed at a position where the cleaning member (180) contacts with the gap forming surfaces (61c, 62a) when the sensor side portion and the cleaning member side portion move relatively
   wherein the machine (131) is configured such that when the cleaning member side portion is rotated relative to the sensor side portion at which the lubricant deterioration sensor (137, 139) is mounted, the cleaning member (180) is inserted into the oil gap (60a) of the gap forming member (60) and moves in the rotation direction of the cleaning member side portion through the oil gap (60a) so as to contact with the gap forming surfaces (61c, 62a).

2. The machine (131) according to claim 1, wherein the cleaning member (180) includes a plurality of insertion portions (181) configured to be inserted in the oil gap (60a) and having elasticity,
   a width (181a) of each of the insertion portions (180) in a distance direction of the oil gap (60a) is smaller than the distance of the oil gap (60a), and
   an entire width of the plurality of insertion portions (180) in the distance direction of the oil gap (60a) is larger than the distance of the oil gap (60a).

3. The machine (131) according to claim 1 or 2, wherein the lubricant deterioration sensor (137, 139) includes: a supporting member (30) supporting the light emitting element (72), the color light reception element (73) and the gap forming member (60); and a fixing member (20) configured to be fixed to the machine body (132), and
   the fixing member (20) supports the supporting member (30) so as to be rotatable so that a direction of an opening of the oil gap (60a) changes when the supporting member (30) rotates relative to the fixing member (20) which is fixed to the machine body (132).

4. The machine (131) according to claim 3, wherein the supporting member (30) includes a rotational driving force receiving portion (51), which receives a rotational driving force with respect to the fixing member (20) from outside by a contact force, at a position where the rotational driving force receiving portion (51) does not contact with the lubricant in a state where the fixing member (20) is fixed to the machine body (132).

5. The machine (131) according to claim 3 or 4, wherein the lubricant deterioration sensor (137, 139) includes a rotation preventing member (12) preventing a rotation of the supporting member (30) with respect to the fixing member (20) by contacting with both the supporting member (30) and the fixing member (20), and the rotation preventing member (12) includes a contact driving force receiving portion (12a), for receiving a driving force for contacting with both the supporting member (30) and the fixing member (20) from outside by a contact force, at a position where the contact driving force receiving portion (12a) does not contact with the lubricant in a state where the fixing member (20) is fixed to the machine body (132).

6. The machine (131) according to any one of claims 1 to 5, which is adapted to contain a lubricant (131a) that does not contain molybdenum as additive.

7. The machine (131) according to any one of claims 1 to 6, wherein
the light emitting element (72) is a white LED which emits white light.

8. The machine (131) according to any one of claims 1 to 7, wherein
the gap forming member (60) has a reflection surface (61b, 62b) for bending
the optical path.

9. The machine (131) according to claim 8, wherein
the gap forming member (60) includes two right-angle prisms (61, 62) each provided with the reflection surface (61b, 62b) for bending the optical path by 90 degrees, the optical path being bent by 180 degrees by the reflection surfaces (61b, 62b) of the two right-angle prisms (61, 62), and
the oil gap (60a) is formed between the two right-angle prisms (61, 62).

10. The machine (131) according to any one of claims 3 to 9, wherein
the supporting member (30) has an optical path surrounding portion (40) which surrounds at least a part of the optical path, and
the optical path surrounding portion (40) has a surface which is treated for preventing light reflection. -

11. The machine (131) according to any one of claims 1 to 10, wherein
the gap forming surfaces (61b, 62b) are treated by an oil repelling treatment.

12. The machine (131) according to any one of claims 1 to 11, wherein
the machine (131) is a reduction gear for an industrial robot, and
the machine body is a main body of the reduction gear.

13. The machine (131) according to any one of claims 1 to 11, wherein
the machine is an industrial robot,
the machine body includes an arm and a reduction gear used at an articular portion of the arm, and
the lubricant is lubricant for the reduction gear.

14. The machine according to claim 13, wherein
the arm is the sensor side portion,
the reduction gear is the cleaning member side portion, and
the cleaning member is disposed at a position contacting with the gap forming surfaces when the reduction gear rotates.

## Patentansprüche

1. Eine Maschine (131), die konfiguriert ist, dass sie eine Verschlechterung eines Schmiermittels erkennt, enthaltend:

einen Maschinenkörper (132);
einen Schmiermittelverschlechterungssensor (137, 139), der konfiguriert ist, dass er eine Verschlechterung des Schmiermittels erfasst, wobei das Schmiermittel dazu dient, die an einem beweglichen Abschnitt des Maschinenkörpers (132) erzeugte Reibung zu verringern; und
ein Reinigungselement (180), das zur Reinigung des Schmiermittelverschlechterungssensors (137, 139) konfiguriert ist,
wobei
der Maschinenkörper (132) einen Sensorseitenabschnitt, an dem der Schmiermittelverschlechterungssensor (137, 139) angebracht ist, und einen Reinigungselementseitenabschnitt, der in Bezug auf den Sensorseitenabschnitt beweglich ist und an dem das Reinigungselement (180) angebracht ist, umfasst,
der Schmiermittelverschlechterungssensor ein lichtemittierendes Element (72) umfasst, das konfiguriert ist, dass es Licht emittiert, ein Farblichtempfangselement (73), das konfiguriert ist, dass es die Farbe des empfangenen Lichts erfasst, und ein spaltbildendes Element (60) mit spaltbildenden Oberflächen (61c, 62a), an denen ein Ölspalt (60a) gebildet wird, in den das Schmiermittel (131a) eintritt, enthält,
das spaltbildende Element (60) konfiguriert ist, dass es das von dem lichtemittierenden Element (72) emittierte Licht durchlässt,
der Ölspalt (60a) auf einem optischen Weg vom lichtemittierenden Element (72) zum Farblichtempfangselement (73) angeordnet ist, und
das Reinigungselement (180) an einer Position angeordnet ist, an der das Reinigungselement (180) mit den spaltbildenden Oberflächen (61c, 62a) in Kontakt kommt, wenn sich der Sensorseitenabschnitt und der Reinigungselementseitenabschnitt relativ zueinander bewegen
wobei die Maschine (131) so konfiguriert ist, dass, wenn der Reinigungselementseitenabschnitt relativ zu dem Sensorseitenabschnitt, an dem der Schmiermittelverschlechterungssensor (137, 139) angebracht ist, gedreht wird, das Reinigungselement (180) in den Ölspalt (60a) des spaltbildenden Elements (60) eingeführt wird und sich in der Drehrichtung des Reinigungselementseitenabschnitts durch den Ölspalt (60a) bewegt, so dass es mit den spaltbildenden Oberflächen (61c, 62a) in Kontakt kommt.

2. Die Maschine (131) nach Anspruch 1, wobei das Reinigungselement (180) eine Vielzahl von Einführungsabschnitten (181) aufweist, die so konfiguriert sind, dass sie in den Ölspalt (60a) eingeführt werden können und eine Elastizität aufweisen, eine Breite (181a) jedes der Einführungsabschnitte (180) in einer Abstandsrichtung des Ölspalts (60a) kleiner ist als der Abstand des Ölspalts (60a), und eine gesamte Breite der Vielzahl von Einführungsabschnitten (180) in der Abstandsrichtung des Ölspalts (60a) größer ist als der Abstand des Ölspalts (60a).

3. Die Maschine (131) nach Anspruch 1 oder 2, wobei der Schmiermittelverschlechterungssensor (137, 139) umfasst: ein Stützelement (30), das das lichtemittierende Element (72), das Farblichtempfangselement (73) und das spaltbildende Element (60) trägt; und ein Befestigungselement (20), das so konfiguriert ist, dass es am Maschinenkörper (132) befestigt werden kann, und das Befestigungselement (20) das Stützelement (30) so stützt, dass es drehbar ist, so dass sich eine Richtung einer Öffnung des Ölspalts (60a) ändert, wenn sich das Stützelement (30) relativ zu dem Befestigungselement (20), das an dem Maschinenkörper (132) befestigt ist, dreht.

4. Die Maschine (131) nach Anspruch 3, wobei das Stützelement (30) einen Drehantriebskraft-Aufnahmeabschnitt (51) aufweist, der eine Drehantriebskraft in Bezug auf das Befestigungselement (20) von außen durch eine Kontaktkraft an einer Position aufnimmt, an der der Drehantriebskraft-Aufnahmeabschnitt (51) in einem Zustand, in dem das Befestigungselement (20) an dem Maschinenkörper (132) befestigt ist, nicht mit dem Schmiermittel in Kontakt kommt.

5. Die Maschine (131) nach Anspruch 3 oder 4, wobei der Schmiermittelverschlechterungssensor (137, 139) ein Drehverhinderungselement (12) aufweist, das eine Drehung des Stützelements (30) in Bezug auf das Befestigungselement (20) durch Kontakt sowohl mit dem Stützelement (30) als auch mit dem Befestigungselement (20) verhindert, und das Drehverhinderungselement (12) einen Kontaktantriebskraft-Aufnahmeabschnitt (12a) zum Aufnehmen einer Antriebskraft zum Kontaktieren sowohl des Stützelements (30) als auch des Befestigungselements (20) von außen durch eine Kontaktkraft an einer Position, an der der Kontaktantriebskraft-Aufnahmeabschnitt (12a) in einem Zustand, in dem das Befestigungselement (20) an dem Maschinenkörper (132) befestigt ist, nicht mit dem Schmiermittel in Kontakt kommt, umfasst.

6. Die Maschine (131) nach einem der Ansprüche 1 bis 5, wobei diese so konfiguriert ist dass sie ein Schmiermittel (131a) enthält, welches kein Molybdän als Additiv enthält.

7. Die Maschine (131) nach einem der Ansprüche 1 bis 6, wobei das lichtemittierende Element (72) eine weiße LED ist, die weißes Licht ausstrahlt.

8. Die Maschine (131) nach einem der Ansprüche 1 bis 7, wobei das spaltbildende Element (60) eine Reflexionsfläche (61b, 62b) zum Biegen des optischen Pfades aufweist.

9. Die Maschine (131) nach Anspruch 8, wobei das spaltbildende Element (60) zwei rechtwinklige Prismen (61, 62) enthält, die jeweils mit der Reflexionsfläche (61b, 62b) zum Biegen des optischen Weges um 90 Grad versehen sind, wobei der optische Weg durch die Reflexionsflächen (61b, 62b) der beiden rechtwinkligen Prismen (61, 62) um 180 Grad gebogen wird, und der Ölspalt (60a) zwischen den beiden rechtwinkligen Prismen (61, 62) gebildet wird.

10. Die Maschine (131) nach einem der Ansprüche 3 bis 9, wobei das Stützelement (30) einen den optischen Pfad umgebenden Abschnitt (40) aufweist, der zumindest einen Teil des optischen Pfades umgibt, und der den optischen Pfad umgebenden Abschnitt (40) eine Oberfläche aufweist, die zur Verhinderung von Lichtreflexionen behandelt ist.

11. Die Maschine (131) nach einem der Ansprüche 1 bis 10, wobei die spaltbildenden Oberflächen (61b, 62b) mit einer ölabweisenden Behandlung behandelt sind.

12. Die Maschine (131) nach einem der Ansprüche 1 bis 11, wobei die Maschine (131) ist ein Untersetzungsgetriebe für einen Industrieroboter ist, und der Maschinenkörper ein Hauptkörper des Untersetzungsgetriebes ist.

13. Die Maschine (131) nach einem der Ansprüche 1 bis 11, wobei die Maschine ein Industrieroboter ist, der Maschinenkörper einen Arm und ein Untersetzungsgetriebe enthält, das an einem Gelenkabschnitt des Arms verwendet wird, und das Schmiermittel ein Schmiermittel für das Untersetzungsgetriebe ist.

14. Die Maschine nach Anspruch 13, wobei

der Arm der Sensorseitenabschnitt ist,
das Untersetzungsgetriebe ist der Reinigungselementseitenabschnitt ist, und
das Reinigungselement an einer Stelle angeordnet ist, die mit den spaltbildenden Flächen in Kontakt steht, wenn sich das Untersetzungsgetriebe dreht.

## Revendications

1. Machine (131) configurée pour détecter une détérioration de lubrifiant, comprenant :

un corps de machine (132) ;
un capteur de détérioration de lubrifiant (137, 139) qui est configuré pour détecter une détérioration du lubrifiant, lequel lubrifiant est destiné à réduire un frottement généré au niveau d'une partie mobile du corps de machine (132) ; et
un élément de nettoyage (180) qui est configuré pour nettoyer le capteur de détérioration de lubrifiant (137, 139),
dans laquelle
le corps de machine (132) comprend une partie latérale de capteur sur laquelle le capteur de détérioration de lubrifiant (137, 139) est monté et une partie latérale d'élément de nettoyage qui est mobile par rapport à la partie latérale de capteur et sur laquelle l'élément de nettoyage (180) est monté,
le capteur de détérioration de lubrifiant comprend un élément émetteur de lumière (72) qui est configuré pour émettre de la lumière, un élément de réception de lumière colorée (73) qui est configuré pour détecter une couleur de lumière reçue, et un élément de formation d'espace (60) ayant des surfaces de formation d'espace (61c, 62a) au niveau duquel est formé un espace d'huile (60a) dans lequel pénètre le lubrifiant (131a),
l'élément de formation d'espace (60) est configuré pour transmettre la lumière émise par l'élément émetteur de lumière (72),
l'espace d'huile (60a) est disposé sur un chemin optique allant de l'élément émetteur de lumière (72) jusqu'à l'élément de réception de lumière colorée (73), et
l'élément de nettoyage (180) est disposé à une position où l'élément de nettoyage (180) entre en contact avec les surfaces de formation d'espace (61c, 62a) lorsque la partie latérale de capteur et la partie latérale d'élément de nettoyage se déplacent de manière relative
dans laquelle la machine (131) est configurée de telle sorte que, lorsque la partie latérale d'élément de nettoyage est tournée par rapport à la partie latérale de capteur sur laquelle le capteur de détérioration de lubrifiant (137, 139) est monté, l'élément de nettoyage (180) est inséré dans l'espace d'huile (60a) de l'élément de formation d'espace (60) et se déplace dans le sens de rotation de la partie latérale d'élément de nettoyage à travers l'espace d'huile (60a) de manière venir au contact des surfaces de formation d'espace (61c, 62a).

2. Machine (131) selon la revendication 1, dans laquelle
l'élément de nettoyage (180) comprend une pluralité de parties d'insertion (181) configurées pour être insérées dans l'espace d'huile (60a) et ayant une élasticité,
une largeur (181a) de chacune des parties d'insertion (180) dans une direction de distance de l'espace d'huile (60a) est inférieure à la distance de l'espace d'huile (60a), et
une largeur entière de la pluralité de parties d'insertion (180) dans la direction de distance de l'espace d'huile (60a) est supérieure à la distance de l'espace d'huile (60a).

3. Machine (131) selon la revendication 1 ou 2, dans laquelle
le capteur de détérioration de lubrifiant (137, 139) comprend : un élément de support (30) supportant l'élément émetteur de lumière (72), l'élément de réception de lumière colorée (73) et l'élément de formation d'espace (60) ; et un élément de fixation (20) configuré pour être fixé au corps de machine (132), et
l'élément de fixation (20) supporte l'élément de support (30) de manière à pouvoir tourner de sorte qu'une direction d'une ouverture de l'espace d'huile (60a) change lorsque l'élément de support (30) tourne par rapport à l'élément de fixation (20) qui est fixé au corps de machine (132).

4. Machine (131) selon la revendication 3, dans laquelle
l'élément de support (30) comprend une partie de réception de force d'entraînement en rotation (51), qui reçoit une force d'entraînement en rotation par rapport à l'élément de fixation (20) depuis l'extérieur par une force de contact, à une position où la partie de réception de force d'entraînement en rotation (51) n'entre pas en contact avec le lubrifiant dans un état où l'élément de fixation (20) est fixé au corps de machine (132).

5. Machine (131) selon la revendication 3 ou 4, dans laquelle
le capteur de détérioration de lubrifiant (137, 139) comprend un élément de prévention de rotation (12) empêchant une rotation de l'élément de support (30) par rapport à l'élément de fixation (20) en venant au contact à la fois de l'élément de support (30) et de l'élément de fixation (20), et l'élément de prévention de rotation (12) comprend une partie de réception de force d'entraînement de contact (12a), pour recevoir une force d'entraînement pour venir au contact à la fois de l'élément de support (30) et de l'élément de fixation (20) depuis l'extérieur par une force de contact, à une position où la partie de réception de force d'entraînement de contact (12a) n'entre pas en contact avec le lubrifiant dans un état où l'élément de fixation (20) est fixé au corps de machine (132).

6. Machine (131) selon l'une quelconque des revendications 1 à 5, qui est adapté pour contenir un lubrifiant (131a) qui ne contient pas de molybdène comme additif.

7. Machine (131) selon l'une quelconque des revendications 1 à 6, dans laquelle l'élément émetteur de lumière (72) est une LED blanche qui émet de la lumière blanche.

8. Machine (131) selon l'une quelconque des revendications 1 à 7, dans laquelle l'élément de formation d'espace (60) a une surface de réflexion (61b, 62b) pour incurver le chemin optique.

9. Machine (131) selon la revendication 8, dans laquelle l'élément de formation d'espace (60) comprend deux prismes à angle droit (61, 62) munis chacun de la surface de réflexion (61b, 62b) pour incurver le chemin optique de 90 degrés, le chemin optique étant incurvé de 180 degrés par les surfaces de réflexion (61b, 62b) des deux prismes à angle droit (61, 62), et l'espace d'huile (60a) est formé entre les deux prismes à angle droit (61, 62).

10. Machine (131) selon l'une quelconque des revendications 3 à 9, dans laquelle l'élément de support (30) a une partie d'entourage de chemin optique (40) qui entoure au moins une partie du chemin optique, et la partie d'entourage de chemin optique (40) a une surface qui est traitée pour empêcher une réflexion de lumière.

11. Machine (131) selon l'une quelconque des revendications 1 à 10, dans laquelle les surfaces de formation d'espace (61b, 62b) sont traitées par un traitement de répulsion d'huile.

12. Machine (131) selon l'une quelconque des revendications 1 à 11, dans laquelle la machine (131) est un réducteur pour un robot industriel, et le corps de machine est un corps principal du réducteur.

13. Machine (131) selon l'une quelconque des revendications 1 à 11, dans laquelle la machine est un robot industriel, le corps de la machine comprend un bras et un réducteur utilisés au niveau d'une partie d'articulation du bras, et le lubrifiant est un lubrifiant pour le réducteur.

14. Machine selon la revendication 13, dans laquelle le bras est la partie latérale de capteur, le réducteur est la partie latérale d'élément de nettoyage, et l'élément de nettoyage est disposé à une position en contact avec les surfaces de formation d'espace lorsque le réducteur tourne.

*Fig. 1*

*Fig. 2*

*Fig. 3*

*Fig. 4*

## Fig. 5

(a)

(b)

*Fig. 6*

( a )

( b )

*Fig. 7*

( a )

( b )

*Fig. 8*

( a )

( b )

Fig. 9

40

49

(a)

40

48                48

47            47

44            43

46

49

45

42        41
42a       41a

(b)

*Fig. 10*

40

49

41

41a

( a )

40

48

48

47

47

49

42

42a

( b )

*Fig. 11*

( a )

( b )

*Fig. 12*

*Fig. 13*

50

(a)

50

52   51

53   53

(b)

*Fig. 14*

(a)

(b)

*Fig. 15*

Fig. 16

180

181

（a）

180

181

（b）

*Fig. 17*

(a)

(b)

Fig. 18

## Fig. 19

LUBRICANT CONTAINING Mo-BASED ADDITIVE

| RELATED CONVERSION TIME | COLOR DIFFERENCE $\Delta E$ | |
|---|---|---|
| | SAMPLING MEASUREMENT | REAL TIME MEASUREMENT |
| 0 | 360.0 | 358.0 |
| 254 | 334.0 | 308.0 |
| 358 | 328.0 | 293.0 |
| 616 | 317.0 | 252.0 |
| 875 | 311.0 | 213.0 |

(a)

(b)

*Fig. 20*

LUBRICANT NON CONTAINING Mo-BASED ADDITIVE

| RELATED CONVERSION TIME | COLOR DIFFERENCE $\Delta E$ | |
|---|---|---|
| | SAMPLING MEASUREMENT | REAL TIME MEASUREMENT |
| 0 | 440.6 | 438.2 |
| 233 | 421.8 | 425.2 |
| 669 | 428.2 | 408.0 |
| 929 | 425.7 | 410.0 |

( a )

( b )

*Fig. 21*

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 20090285721 A1
- WO 9816822 A
- JP 2008215987 A
- JP 7146233 A
- JP 10104160 A

## Non-patent literature cited in the description

- METHOD OF DISCRIMINATING HUE OF CONTAMINANT WITHIN LUBRICANT. **TOMOHIKO YAMAGUCHI.** Faculty of Engineering, Research Report. Fukui University, March 2003, vol. 51, 81-88
- **TOMOMI HONDA.** DETERIORATION DIAGNOSIS OF LUBRICANT ·INSPECTION TECHNOLOGY. *Journal of Japan Society for Precision Engineering,* 2009, vol. 75 (3), 359-362
- RGB sensor. Hamamatsu Photonics K.K, vol. 73, S9032-02